# EUROPÄISCHE PATENTSCHRIFT

(11) **EP 2 346 885 B1**
(45) Veröffentlichungstag und Bekanntmachung des Hinweises auf die Patenterteilung: **28.08.2013**
(21) Anmeldenummer: 09778827.7
(22) Anmeldetag: 06.10.2009
(51) Int. Cl.: C07F 9/30, C07F 9/48, C08K 5/53, C09K 21/12

(54) **VERFAHREN ZUR HERSTELLUNG VON MONOCARBOXYFUNKTIONASLISIERTEN DIALKYLPHOSPHINSÄUREN, -ESTERN UND -SALZEN MITTELS ALLYLALKOHOLEN/ACROLEINEN UND IHRE VERWENDUNG**
METHOD FOR PRODUCING MONO-CARBOXY-FUNCTIONALIZED DIALKYLPHOSPHINIC ACIDS AND ESTERS AND SALTS THEREOF BY MEANS OF ALLYL ALCOHOLS/ACROLEINS AND USE THEREOF
PROCÉDÉ DE PRODUCTION D'ACIDES, D'ESTERS ET DE SELS PHOSPHINIQUES DIALKYLE À FONCTIONNALISATION MONOCARBOXY, AU MOYEN D'ALCOOLS ALLYLIQUES/ACROLÉINES ET LEUR UTILISATION

(30) Priorität: 05.11.2008 DE 102008055915
(43) Veröffentlichungstag der Anmeldung: 27.07.2011
(73) Patentinhaber: Clariant International Ltd., 4132 Muttenz 1 (CH)
(72) Erfinder: HILL, Michael, 22043 Hamburg (DE); KRAUSE, Werner, 50354 Hürth (DE); SICKEN, Martin, 51149 Köln (DE)
(74) Vertreter: Mikulecky, Klaus
(86) Internationale Anmeldenummer: PCT/EP2009/007123
(87) Internationale Veröffentlichungsnummer: WO 2010/051883

(56) Entgegenhaltungen:
- EP-A1- 1 832 594
- EP-A1- 1 832 595
- EP-A1- 1 832 596
- US-A- 4 594 199
- US-B1- 6 384 022
- MONTCHAMP J L: "Recent advances in phosphorus-carbon bond formation: synthesis of H-phosphinic acid derivatives from hypophosphorous compounds" JOURNAL OF ORGANOMETALLIC CHEMISTRY, ELSEVIER-SEQUOIA S.A. LAUSANNE, CH, Bd. 690, Nr. 10, 16. Mai 2005 (2005-05-16) , Seiten 2388-2406, XP004877374 ISSN: 0022-328X
- SYLVINE DEPRÈLE ET AL: "Palladium-Catalyzed Hydrophosphinylation of Alkenes and Alkynes" JOURNAL OF THE AMERICAN CHEMICAL SOCIETY, AMERICAN CHEMICAL SOCIETY, WASHINGTON, DC. US, Bd. 124, Nr. 32, 1. Januar 2002 (2002-01-01), Seite 9387, XP002500862 ISSN: 0002-7863
- BRAVO-ALTAMIRANO ET AL: "A novel approach to phosphonic acids from hypophosphorous acid" TETRAHEDRON LETTERS, ELSEVIER, AMSTERDAM, NL, Bd. 48, Nr. 33, 19. Juli 2007 (2007-07-19) , Seiten 5755-5759, XP022163552 ISSN: 0040-4039
- SYLVINE DEPRÈLE ET AL: "Environmentally Benign Synthesis of H-Phosphinic Acids Using a Water-Tolerant, Recyclable Polymer-Supported Catalyst" ORGANIC LETTERS, AMERICAN CHEMICAL SOCIETY, US, Bd. 6, Nr. 21, 1. Januar 2004 (2004-01-01) , Seiten 3805-3808, XP002500861 ISSN: 1523-7060 [gefunden am 2004-09-18]
- PATRICE RIBIÈRE ET AL: "NiCl2-Catalyzed Hydrophosphinylation" JOURNAL OF ORGANIC CHEMISTRY, AMERICAN CHEMICAL SOCIETY, EASTON.; US, Bd. 70, Nr. 10, 1. Januar 2005 (2005-01-01), Seiten 4064-4072, XP002530191 ISSN: 0022-3263
- COUDRAY L. ET AL.: "Allylic Phosphinates via Pd-Catalyzed Allylation of H-Phosphinic Acids with Allylic Alcohols" ORGANIC LETTERS, Bd. 10, Nr. 6, 21. Februar 2008 (2008-02-21), Seiten 1123-1126, XP002561368

## Beschreibung

Die Erfindung betrifft ein Verfahren zur Herstellung von Dialkylphosphinsäuren, -estern und -salzen sowie ihre Verwendung.

Von bestimmten Dialkylphosphinsäuren, den sog. mono-carboxyfunktionalisierten Dialkylphosphinsäuren, wie sie weiter unten definiert sind, sind bisher fast ausschließlich die Ester zugänglich. Letztere können über mehrere Schritte ausgehend von Phosphonigsäure-dihalogeniden hergestellt werden. Hierzu gehört die Umsetzung von Dihalogenphosphinen mit aktivierten olefinischen Verbindungen wie Acrylsäure, gefolgt von der Veresterung der zunächst gebildeten Säurechlorid- und Anhydrid-Derivate mit Alkoholen (V.K. Khairullin, R. R. Shagidullin, Zh. Obshch. Khim. 36, 289-296).

Dialkylphosphinsäuren im Sinne der vorliegenden Erfindung sind also immer mono-carboxyfunktionalisierten Dialkylphosphinsäuren, auch wenn dies nicht ausdrücklich erwähnt ist. Dies schließt die entsprechenden Ester und Salze mit ein.

Solche Dialkylphosphinsäureester werden auch erhalten, wenn man Phosphonigsäureester in Gegenwart peroxidischer Katalysatoren an α,β-ungesättigte Carbonsäureester addiert (Houben-Weyl, Band 1211, S. 258-259). Die Phosphonig-säureester selbst werden aus Phosphonigsäuredihalogeniden durch Umsetzung mit Alkoholen bzw. Hydrolyse und anschließender Veresterung hergestellt. Die vorgenannten Phosphonigsäuredihalogenide selbst werden in einer aufwendigen Synthese aus Phosphortrichlorid und Alkylchlorid in Gegenwart von Aluminiumchlorid hergestellt (Houben-Weyl, Band 1211, S. 306). Die Reaktion ist stark exotherm und technisch nur schwierig zu beherrschen. Es werden zudem verschiedene Nebenprodukte gebildet, die, wie zum Teil auch die vorgenannten Ausgangsprodukte, giftig undloder korrosiv, also höchst unerwünscht sind (insbesondere weil die Produkte nicht halogenfrei hergestellt werden können).

Dialkylphosphinsäuren sind Gegenstand verschiedener Abhandlungen, so beschreibt J.L. Montchamp in Journal of Organometallic Chemistry, Bd. 690, Nr. 10, 16.05.2005, Seiten 2388 - 2406 die Synthese von Phosphinsäurederivaten, ausgehend von hypophosphorigen Verbindungen. Dabei werden - Palladiumkatalysiert - in einem flüssigen System Olefine mit mindestens 8 C-Atomen an die hypophosphorige Verbindung addiiert.

Ähnlich wird nach S. Deprele et al., J. Am. Chem. Soc., Bd. 124, Nr. 32, 01.01.2002, Seite 9387, hypophosphorige Säure in wässriger Form mit Olefinen, die mindestens 8 C-Atome aufweisen oder mit Alkinen, die ebenfalls mindestens 8 C-Atome aufweisen zu den entsprechenden P-Verbindungen, die dann ebenfalls mindestens 8 C-Atome enthalten, umgesetzt.

In Tetrahedron Lett., Bd. 48, Nr. 33, 19.07.2007, Seiten 5755 - 5759 (Bravo-Altamirano et al.) wird die Palladium-katalysierte Herstellung von allylgruppenhaltigen Phosphinsäuren beschrieben, bei dem Alkine bzw. Alkene mit mehr als 7 C-Atomen mit phosphoriger Säure umgesetzt werden.

Die Reaktion von ausschließlich hypophosphoriger Säure in flüssiger Form mit Kohlenstoffverbindungen, die mindestens 8 C-Atome aufweisen und eine C = C- oder C = C-Verbindung enthalten, wird durch S. Deprele et al., in Org. Lett., Bd. 6, Nr. 21, 01.01.2004, Seiten 3805 - 3808 beschrieben. Erhalten werden P-Verbindungen, die eine Alkylgruppe mit mindestens 8 C-Atomen aufweisen.

P. Ribiere et al., J. Org. Chem., Bd. 70, Nr. 10, 01.01.2005, Seiten 4064 - 4072 beschreiben die Nickel-katalysierte Hydrophosphorylierung von Phosphinsäuren mit verschiedenen Olefinen, insbesondere mit 1-Octen sowie die Synthese von Alkenyl-H-Phosphinaten, die eine C = C-Bindung aufweisen.

Die Umsetzung von Phosphinsäuren mit Allyl-Verbindungen zu den entsprechenden allylischen Phosphinaten wird beschrieben in Org. Lett., Bd. 10, Nr. 6, 06.02.2008, Seiten 1123 - 1126 (Autor: L. Condray et al.).

Die US-A-4594199 beschreibt ein Verfahren zur Herstellung von Phosphinsäure-Prodrug-Zwischenprodukten, die für die Herstellung von Phosphinsäure-Angiotensin-Converting-Enzyminhibitoren geeignet sind.

Ein weiteres Verfahren zur Herstellung von mono-carboxyfunktionalisierten Dialkylphosphinsäureestern basiert auf der Umsetzung von gelbem Phosphor mit Methylchlorid, wobei Methylphosphonigsäure entsteht, die in dann verestert und daraufhin mit Acrylsäureester umgesetzt wird (DE-A-101 53 780).

Mono-carboxyfunktionalisierte Dialkylphosphinsäureester können auch durch Umsetzung von Phosphonigsäure- Bis(trimethylsilyl)ester - HP(OSiMe₃)₂ - mit α,ß-ungesättigten Carbonsäure-Komponenten, anschließender Alkylierung mit Alkylhalogeniden nach der Arbuzov-Reaktion und Alkoholyse erhalten werden (Kurdyumova, N. R.; Rozhko, L. F.; Ragulin, V. V.; Tsvetkov, E. N.; Russian Journal of General Chemistry (Translation of Zhumal Obshchei Khimii (1997), 67(12), 1852-1856). Der Phosphonigsäure-Bis(trimethylsilyl)ester wird dabei aus Kalium- oder Ammoniumhypophosphit durch Umsetzung mit Hexamethyldisilazan erhalten.

Bisher fehlt es an Verfahren zur Herstellung von mono-carboxyfunktionalisierten Dialkylphosphinsäuren, -estern und -salzen, die wirtschaftlich und großtechnisch zugänglich sind und die insbesondere eine hohe Raum-/Zeitausbeute ermöglichen. Auch fehlt es an Verfahren, die ohne störende Halogenverbindungen als Edukte ausreichend effektiv sind und zudem an solchen, bei denen die Endprodukte leicht erhalten bzw. isoliert werden können oder unter gezielten Reaktionsbedingungen (wie etwa einer Umesterung) gezielt und gewünscht hergestellt werden können.

Diese Aufgabe wird gelöst durch ein Verfahren zur Herstellung von mono-carboxyfunktionalisierten Dialkylphosphinsäuren, -estern und -salzen, dadurch gekennzeichnet, dass man
a) eine Phosphinsäurequelle (I) 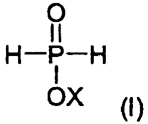 mit Olefinen (IV) 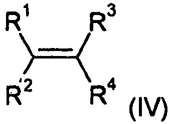 in Gegenwart eines Katalysators A zu einer Alkylphosphonigsäure, deren Salz oder Ester (II) 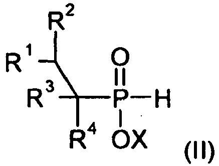 umsetzt,
b) die so entstandene Alkylphosphonigsäure, deren Salz oder Ester (II) mit einem
   Allylalkohol (V) und/oder Acrolein (V') 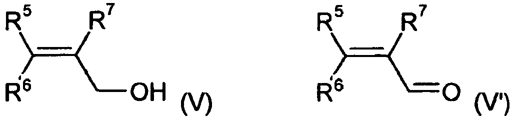 in Gegenwart eines Katalysators B zum mono-funktionalisierten Dialkylphosphinsäurederivat (VI) und/oder (VI') umsetzt und 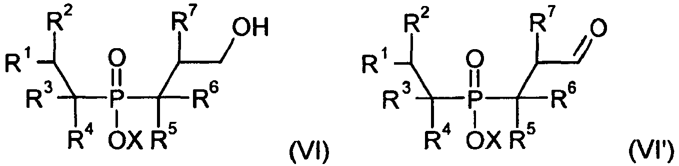
c) das mono-funktionalisierten Dialkylphosphinsäurederivat (VI) und/oder (VI') mit einem Oxidationsmittel oder mit einem Oxidationsmittel und Wasser oder in Gegenwart eines Katalysators C mit Sauerstoff und Wasser zum mono-carboxyfunktionalisierten Dialkylphosphinsäurederivat (III) 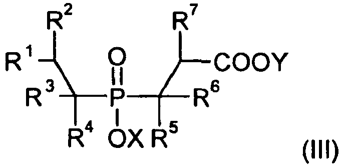 umsetzt oder die nach Schritt a) erhaltene Alkylphosphonigsäure, deren Salz oder Ester (II) und/oder die nach Schritt b) erhaltene mono-funktionalisierte Dialkylphosphinsäure, deren Salz oder Ester (VI) und/oder (VI') und/oder die nach Schritt c) erhaltene mono-carboxyfunktionalisierte Dialkylphosphinsäure, deren Salz oder Ester (III) und/oder die jeweils resultierende Reaktionslösung davon mit einem Alkylenoxid oder einem Alkohol M-OH und/oder M'-OH verestert, und den jeweils entstandenen Alkylphosphonigsäureester (II), monofunktionalisierten Dialkylphosphinsäurester (VI) und/oder (VI') und/oder mono-carboxyfunktionalisierten Dialkylphosphinsäureester (III) den weiteren Reaktionsschritten b) oder c) unterwirft, wobei R¹, R², R³, R⁴, R⁵, R⁶, R⁷ gleich oder verschieden sind und unabhängig voneinander H, C₁-C₁₈-Alkyl, C₆-C₁₈₋Aryl, C₆-C₁₈-Aralkyl, C₆-C₁₈-Alkyl-Aryl, CN, CHO, OC(O)CH₂CN, CH(OH)C₂H₅, CH₂CH(OH)CH₃, 9-Anthracen, 2-Pyrrolidon, (CH₂)mOH, (CH₂)ₘNH₂, (CH₂)ₘNCS, (CH₂)ₘNC(S)NH₂, (CH₂)ₘSH, (CH₂)ₘS-2-thiazolin, (CH₂)ₘSiMe₃, C(O)R⁸, CH=CH-R⁸, CH=CH-C(O)_{R}⁸ bedeuten, wobei die Gruppen C₆-C₁₈-Aryl, C₆-C₁₈-Aralkyl und C₆-C₁₈-Alkyl-Aryl mit -C(O)CH₃, OH, CH₂OH, NH₂, NO₂, OCH₃, SH und/oder OC(O)CH₃ substituiert sein können und wobei R⁸ für C₁-C₈-Alkyl oder C₆-C₁₈-Aryl steht und m eine ganze Zahl von 0 bis 10 bedeutet und X und Y gleich oder verschieden sind und unabhängig voneinander für H, C₁-C₁₈-Alkyl, C₆-C₁₈-Aryl, C₆-C₁₈-Aralkyl, C₆-C₁₈-Alkyl-Aryl, (CH₂)ₖOH, CH₂-CHOH-CH₂OH, (CH₂)ₖO(CH₂)ₖH, (CH₂)ₖ-CH(OH)-(CH₂)ₖH, (CH₂-CH₂O)ₖH, (CH₂-C[CH₃]HO)ₖH, (CH₂-C[CH₃]HO)ₖ(CH₂-CH₂O)kH, (CH₂-CH₂O)ₖ(CH₂-C[CH₃]HO)H, (CH₂-CH₂O)ₖ-alkyl, (CH₂-C[CH₃]HO)ₖ-alkyl, (CH₂-C[CH₃]HO)ₖ(CH₂-CH₂O)ₖ-alkyl, (CH₂-CH₂O)ₖ(CH₂-C[CH₃]HO)O-alkyl, (CH₂)ₖ-CH=CH(CH₂)ₖH, (CH₂)ₖNH₂, (CH₂)ₖN[(CH₂)ₖH]₂ stehen wobei k eine ganze Zahl von 0 bis 10 ist und/oder für Mg, Ca, Al, Sb, Sn, Ge, Ti, Fe, Zr, Zn, Ce, Bi, Sr, Mn, Cu, Ni, Li, Na, K, H und/oder eine protonierte Stickstoffbase steht und es sich bei den Katalysatoren A und C um Übergangsmetalle und/oder Übergangsmetallverbindungen und/oder Katalysatorsysteme handelt, die sich aus einem Übergangsmetall und/oder einer Übergangsmetallverbindung und mindestens einem Liganden zusammensetzen und es sich bei dem Katalysator B um Peroxide bildende Verbindungen und/oder Peroxoverbindungen und/oder um Azo-Verbindungen und/oder um Alkali- und/oder Erdalkalimetalle, -hydride und/oder -alkoholate handelt.

Bevorzugt wird die nach Schritt c) erhaltene mono-carboxyfunktionalisierte Dialkylphosphinsäure, deren Salz oder Ester (III) anschließend in einem Schritt d) mit Metallverbindungen von Mg, Ca, Al, Sb, Sn, Ge, Ti, Fe, Zr, Zn, Ce, Bi, Sr, Mn, Li, Na, K und/oder einer protonierte Stickstoffbase zu den entsprechenden mono-carboxyfunktionalisierten Dialkylphosphinsäuresalzen (III) dieser Metalle und/oder einer Stickstoffverbindung umgesetzt.

Bevorzugt sind R¹, R², R³, R⁴, R⁵, R⁶, R⁷ gleich oder verschieden und bedeuten, unabhängig voneinander H, Methyl, Ethyl, n-Propyl, Isopropyl, n-Butyl, Isobutyl, tert. Butyl und/oder Phenyl.

Bevorzugt sind X und Y gleich oder verschieden und bedeuten jeweils H, Ca, Mg, Al, Zn, Ti, Fe, Ce, Methyl, Ethyl, n-Propyl, Isopropyl, n-Butyl, Isobutyl, tert. Butyl, Phenyl, Ethylenglykol, Propylglykol, Butylglykol, Pentylglykol, Hexylglykol, Allyl und/oder Glycerin.

Bevorzugt handelt es sich bei den Übergangsmetallen und/oder Übergangsmetallverbindungen um solche aus der ersten, siebten und achten Nebengruppe.

Bevorzugt handelt es sich bei den Übergangsmetallen und/oder Übergangsmetallverbindungen um Rhodium, Nickel, Palladium, Platin, Ruthenium und/oder Gold.

Bevorzugt handelt es sich bei dem Katalysator B um Wasserstoffperoxid, Natriumperoxid, Lithiumperoxid, Kaliumpersulfat, Natriumpersulfat, Ammoniumpersulfat, Natriumperoxodisulfat, Kaliumperoxoborat, Peressigsäure, Benzoylperoxid, Di-t-butylperoxid und/oder Peroxodischwefelsäure und/oder um Azodiisobutyronitril, 2,2'-Azobis(2-amidinopropan)-dihydrochlorid und/oder 2,2'-Azobis(N,N'-dimethylen-isobutyramidin)-dihydrochlorid und/oder um Lithium, Lithiumhydrid, Lithium-aluminiumhydrid, Methyllithium, Butyllithium, t-Butyllithium, Lithiumdiisopropylamid, Natrium, Natriumhydrid, Natriumborhydrid, Natriummethanolat, Natriumethanolat oder Natriumbutylat, Kaliummethanolat, Kaliumethanolat und/oder Kaliumbutylat.

Bevorzugt handelt es sich bei den Oxidationsmitteln um Kaliumpermanganat, Braunstein, Chromtrioxid, Kaliumdichromat, Pyridindichromat, Pyridinchlorchromat, Collins-Reagenz, Jones-Reagenz, Corey-Gilman-Ganem-Reagenz, (Dess-Martin-)Periodinan, o-lodoxy-benzoesäure, Rutheniumtetroxid, Rutheniumdioxid, Tetra-n-propyl-perruthenat, Rutheniumtrichlorid/Natriumperiodat, Rutheniumdioxid/Natriumperiodat, Chlor, Hypochlorit und Peroxoverbindungen.

Bevorzugt handelt es sich bei den Allylalkoholderivaten (V) um 2-Propen-1-ol, 2-Methyl-2-propen-1-ol, 2-isobutyl-2-propen-1-ol, 2-(Trimethylsilyl)-2-propen-1-ol, 3-Phenyl-2-propen-1-ol, 3-(Trimethylsilyl)-2-propen-1-ol, 3-(4-Hydroxy-3-methoxyphenyl)-2-propen-1-ol, 2-Methyl-3-phenyl-2-propen-l-ol, 2-Buten-1-ol, 2-Methyl-2-buten-1-ol, 3-(Trimethylsilyl)-2-buten-1-ol, 3-Methyl-2-buten-1-ol, 3-Phenyl-2-buten-1-ol, 3-(Trimethylsilyl)-2-buten-1-ol, 2-Methyl-3-phenyl-2-buten-1-ol, 2-Penten-1-ol, 2-Methyl-2-penten-1-ol, 2-(Trimethylsilyl)-2-penten-1-ol, 3-Methyl-2-penten-1-ol, 3-Phenyl-2-penten-1-ol, 4-Methyl-2-penten-1-ol und/oder 4-Phenyl-2-penten-1-ol.

Bevorzugt handelt es sich bei den Acroleinderivaten (V') um 2-Propenal, 2-Methyl-2-propenal, 2-Phenyl-2-propenal, 3-Phenyl-2-propenal, 2-Methyl-3-phenyl-2-propenal, 2-Butenal, 2-Methyl-2-butenal, 2-Phenyl-2-butenal, 3-Methyl-2-butenal, 2-Methyl-2-butenal, 2-Pentenal, 2-Methyl-2-pentenal, 2-Phenyl-2-pentenal, 4-Methyl-2-phenyl-2-pentenal und/oder 2,2-Dimethyl-4-pentenal.

Bevorzugt handelt es bei dem Alkohol der allgemeinen Formel M-OH um lineare oder verzweigte, gesättigte und ungesättigte, einwertige organische Alkohole mit einer Kohlenstoffkettenlänge von C₁-C₁₈ und es bei dem Alkohol der allgemeinen Formel M'-OH um lineare oder verzweigte, gesättigte und ungesättigte, mehrwertige organische Alkohole mit einer Kohlenstoffkettenlänge von C₁-C₁₈. Die Erfindung betrifft zudem die Herstellung von mono-carboxyfunktionalisierten Dialkylphosphinsäuren, -salzen und -estern, nach einem oder mehreren der Ansprüche 1 bis 11 und anschließende Verwendung dieser Produkte als Binder, als Vernetzer bzw. Beschleuniger beim Aushärten von Epoxyharzen, Polyurethanen, ungesättigten Polyesterharzen, als Polymerstabilisatoren, als Pflanzenschutzmittel, als Sequestrierungsmittel, als Mineralöl-Additiv, als Korrosionsschutzmittel, in Wasch- und Reinigungsmittelanwendungen, in Elektronikanwendungen.

Bevorzugt wird das Katalysatorsystem A bzw. C durch Umsetzung von einem Übergangsmetall und/oder einer Übergangsmetallverbindung und mindestens einem Liganden gebildet.

Die Erfindung betrifft ebenfalls die Verwendung von mono-carboxyfunktionalisierte Dialkylphosphinsäuren, -salzen und -estern (III), die nach einem oder mehreren der Ansprüche 1 bis 11 hergestellt wurden, als Flammschutzmittel, insbesondere Flammschutzmittel für Klarlacke und Intumeszenzbeschichtungen, Flammschutzmittel für Holz und andere cellulosehaltige Produkte, als reaktives und/oder nicht reaktives Flammschutzmittel für Polymere, zur Herstellung von flammgeschützten Polymerformmassen, zur Herstellung von flammgeschützten Polymerformkörpern und/oder zum flammhemmend Ausrüsten von Polyester und Cellulose-Rein- und Mischgeweben durch Imprägnierung.

Die Erfindung betrifft auch eine flammgeschützte thermoplastische oder duroplastische Polymerformmasse, enthaltend 0,5 bis 45 Gew.-% mono-carboxyfunktionalisierte Dialkylphosphinsäuren, -salze oder -ester (III), die nach einem oder mehreren der Ansprüche 1 bis 11 hergestellt wurden, 0,5 bis 95 Gew.-% thermoplastisches oder duroplastisches Polymer oder Mischungen derselben, 0 bis 55 Gew.-% Additive und 0 bis 55 Gew.-% Füllstoff bzw. Verstärkungsmaterialien, wobei die Summe der Komponenten 100 Gew.-% beträgt.

Schließlich betrifft die Erfindung zudem flammgeschützte thermoplastische oder duroplastische Polymer-Formkörper, -Filme,- Fäden und Fasern, enthaltend 0,5 bis 45 Gew.-% mono-carboxyfunktionalisierte Dialkylphosphinsäuren, -salze oder -ester (III), die nach einem oder mehreren der Ansprüche 1 bis 11 hergestellt wurden, 0,5 bis 95 Gew.-% thermoplastisches oder duroplastisches Polymer oder Mischungen derselben, 0 bis 55 Gew.-% Additive und 0 bis 55 Gew.-% Füllstoff bzw. Verstärkungsmaterialien, wobei die Summe der Komponenten 100 Gew.-% beträgt.

Alle vorgenannten Umsetzungen können auch stufenweise ausgeführt werden; ebenso können in den verschiedenen Verfahrensschritten auch die jeweiligen resultierenden Reaktionslösungen eingesetzt werden.

Handelt es sich bei der mono-carboxyfunktionalisierter Dialkylphosphinsäure (III) nach Schritt d) um einen Ester, so kann bevorzugt eine saure oder basische Hydrolyse durchgeführt werden, um die freie mono-carboxyfunktiönalisierte Dialkylphosphinsäure oder deren Salz zu erhalten.

Bevorzugt handelt es sich bei der mono-carboxyfunktionalisierten Dialkylphosphinsäure um 3-(Ethylhydroxyphosphinyl)-propionsäure, 3-(Propylhydroxyphos-phinyl)-propionsäure, 3-(i-Propylhydroxyphosphinyl)-propionsäure, 3-(Butylhydroxyphosphinyl)-propionsäure, 3-(sec-Butylhydroxyphosphinyl)-propionsäure, 3-(i-Butylhydroxyphosphinyl)-propionsäure, 3-(2-Phenylethylhydroxyphosphinyl)-propionsäure, 3-(Ethylhydroxyphosphinyl)-2-methylpropionsäure, 3-(Propylhydroxyphosphinyl)-2-methylpropionsäure, 3-(i-Propylhydroxyphosphinyl)-2-methylpropionsäure, 3-(Butylhydroxyphosphinyl)-2-methylpropionsäure, 3-(sec-Butylhydroxyphosphinyl)-2-methylpropionsäure, 3-(i-Butylhydroxyphosphinyl)-2-methylpropionsäure, 3-(2-Phenylethylhydroxyphosphinyl)-2-methylpropionsäure, 3-(Ethylhydroxyphosphinyl)-3-phenylpropionsäure, 3-(Propylhydroxyphosphinyl)-3-phenylpropionsäure, 3-(i-Propylhydroxyphosphinyl)-3-phenylpropionsäure, 3-(Butylhydroxyphosphinyl)-3-phenylpropionsäure, 3-(i-Butylhydroxyphosphinyl)-3-phenylpropionsäure, 3-(sec-Butylhydroxyphosphinyl)-3-phenylpropionsäure, 3-(2-Phenylethylhydroxyphosphinyl)-3-phenylpropionsäure.

Bevorzugt handelt es sich bei dem mono-carboxyfunktionalisierten Dialkylphosphinsäureester um einen Propionsäure-, methyl-, ethyl-; i-propyl-; butyl-, phenyl-; 2-hydroxyethyl-, 2-hydroXypropyl-, 3-hydroxypropyl-, 4-hydroxybutyl- und/oder 2,3-dihydroxypropylester der vorgenannten mono-carboxyfunktionalisierten Dialkylphosphinsäuren oder Mischungen davon.

Bevorzugt handelt es sich bei dem mono-carboxyfunktionalisierten Dialkylphosphinsäure-Salz um ein Aluminium(III)-, Calcium(II)-, Magnesium (II)-, Cer(III)-, Ti(IV)- und/oder Zink(II)salz der vorgenannten mono-carboxyfunktionalisierten Dialkylphosphinsäuren oder der vorgenannten Ester der mono-carboxyfunktionalisierten Dialkylphosphinsäuren.

Dabei gelten als Zielverbindungen auch diejenigen Ester und Salze, bei denen die Veresterung bzw. die Salzbildung an der Phosphinsäuregruppe (bei X in Formel (III)) oder an der Propionsäuregruppe (bei Y in Formel (III)) erfolgt.

Bevorzugt handelt es sich bei den Übergangsmetallen für den Katalysator A um Elemente der siebten und achten Nebengruppe (nach moderner Nomenklatur ein Metall der Gruppe 7, 8, 9 oder 10), wie etwa Rhenium, Ruthenium, Cobalt, Rhodium, Iridium, Nickel, Palladium und Platin.

Bevorzugt werden als Quelle der Übergangsmetalle und Übergangsmetallverbindungen deren Metallsalze verwendet. Geeignete Salze sind solche von Mineralsäuren, die die Anionen Fluorid, Chlorid, Bromid, Iodid, Fluorat, Chlorat, Bromat, lodat, Fluorit, Chlorit, Bromit, lodit, Hypofluorit, Hypochlorit, Hypobromit, Hypoiodit, Perfluorat, Perchlorat, Perbromat, Periodat, Cyanid, Cyanat, Nitrat, Nitrid, Nitrit, Oxid, Hydroxid, Borat, Sulfat, Sulfit, Sulfid, Persulfat, Thiosulfat, Sulfamat, Phosphat, Phosphit, Hypophosphit, Phosphid, Carbonat und Sulfonat, wie etwa Methansulfonat, Chlorosulfonat, Fluorosulfonat, Trifluoromethansulfonat, Benzolsulfonat, Naphthyl-sulfonat, Toluolsulfonat, t-Butylsulfonat, 2-Hydroxypropansulfonat und sulfonierte Ionen-tauscherharze; und/oder organische Salze, wie etwa Acetyl-acetonate und Salze einer Carbonsäure mit bis zu 20 Kohlenstoffatomen, wie etwa Format, Acetat, Propionat, Butyrat, Oxalat, Stearat und Zitrat einschliesslich halogenierter Carbonsäuren mit bis zu 20 Kohlenstoffatomen, wie etwa Trifluoracetat, Trichloracetat, enthalten.

Eine weitere Quelle der Übergangsmetalle und Übergangsmetallverbindungen stellen Salze der Übergangsmetalle mit Tetraphenylborat- und halogenierten Tetraphenylboratanionen, wie etwa Perfluorophenylborat, dar.

Geeignete Salze beeinhalten ebenso Doppelsalze und Komplexsalze bestehend aus einem oder mehreren Übergangsmetallionen und unabhängig voneinander ein oder mehrere Alkalimetall-, Erdalkalimetall-, Ammonium-, organische Ammonium-, Phosphonium- und organische Phosphoniumionen und unabhängig voneinander ein oder mehrere oben genannter Anionen. Geeignete Doppelsalze stellen z. B. Ammoniumhexachloropalladat und Ammoniumtetrachloropalladat dar.

Bevorzugt ist eine Quelle der Übergangsmetalle das Übergangsmetall als Element und/oder eine Übergangsmetallverbindung in dessen null-wertigem Zustand.

Bevorzugt wird das Übergangsmetall metallisch eingesetzt oder als Legierung mit weiteren Metallen verwendet, wobei hier Bor, Zirconium, Tantal, Wolfram, Rhenium, Kobalt, Iridium, Nickel, Palladium, Platin und/oder Gold bevorzugt ist. Dabei ist der Übergangsmetallgehalt in der eingesetzten Legierung bevorzugt 45 - 99,95 Gew.-%.

Bevorzugt wird das Übergangsmetall mikrodispers (Teilchengröße 0,1 mm - 100 µm) eingesetzt.

Bevorzugt wird das Übergangsmetall auf einem Metalloxid wie etwa Aluminiumoxid, Siliciumdioxid, Titandioxid, Zirkoniumdioxid, Zinkoxid, Nickeloxid, Vanadiumoxid, Chromoxid, Magnesiumoxid, Celite^{®}, Kieselgur, auf einem Metallcarbonat wie etwa Bariumcarbonat, Calciumcarbonat, Strontiumcarbonat, auf einem Metallsulfat wie etwa Bariumsulfat, Calciumsulfat, Strontiumsulfat, auf einem Metallphosphat wie etwa Aluminiumphosphat, Vanadiumphosphat, auf einem Metallcarbid wie etwa Siliconcarbid, auf einem Metallaluminat wie etwa Calciumaluminat, auf einem Metallsilikat wie etwa Aluminiumsilikat, Kreiden, Zeolithe, Bentonit, Montmorillonit, Hectorit, auf funktionalisierten Silikaten, funktionalisierten Silikagelen wie etwa SiliaBond^{®}, QuadraSil™, auf funktionalisierten Polysiloxanen wie etwa Deloxan^{®}, auf einem Metallnitrid, auf Kohle, Aktivkohle, Mullite, Bauxite, Antimonite, Scheelite, Perovskite, Hydrotalcite, Heteropolyanionen, auf funktionalisierter und unfunktionalisierter Cellulose, Chitosan, Keratin, Heteropolyanionen, auf lonentauschern wie etwa Amberlite™, Amberjet™, Ambersep™, Dowex^{®}, Lewatit^{®}, ScavNet^{®}, auf funktionalisierten Polymeren wie etwa Chelex^{®}, QuadraPure™, Smopex^{®}, PolyOrgs^{®}, auf polymergebundenen Phosphanen, Phosphanoxiden, Phosphinaten, Phosphonaten, Phosphaten, Aminen, Ammoniumsalzen, Amiden, Thioamiden, Harnstoffen, Thioharnstoffen, Triazinen, Imidazolen, Pyrazolen, Pyridinen, Pyrimidinen, Pyrazinen, Thiolen, Thiolether, Thiolester, Alkoholen, Alkoxiden, Ether, Ester, Carbonsäuren, Acetaten, Acetalen, Peptiden, Hetarenen, Polyethylenimin/Siliciumdioxid und/oder Dendrimeren geträgert verwendet.

Geeignete Quellen der Metallsalze und/oder Übergangsmetalle stellen bevorzugt ebenfalls deren Komplexverbindungen dar. Komplexverbindungen der Metallsalze und/oder Übergangsmetalle setzen sich aus den Metallsalzen bzw. Übergangsmetalle und einem oder mehreren Komplexbildnern zusammen. Geeignete Komplexbildner sind z. B. Olefine, Diolefine, Nitrile, Dinitrile, Kohlenmonoxid, Phosphine, Diphosphine, Phosphite, Diphosphite, Dibenzylidenaceton, Cyclopentadienyl, Indenyl oder Styrol. Geeignete Komplexverbindungen der Metallsalze und/oder Übergangsmetalle können auf den oben genannten Trägermaterialien geträgert sein.

Bevorzugt ist der Gehalt an den genannten geträgerten Übergangsmetallen 0,01 bis 20 Gew.-%, vorzugsweise 0,1 bis 10 Gew.-%, insbesondere 0,2 bis 5 Gew.-%, bezogen auf die Gesamtmasse des Trägermaterials.

Geeignete Quellen von Übergangsmetallen und Übergangsmetallverbindungen sind beispielsweise Palladium, Platin, Nickel, Rhodium; Palladium Platin, Nickel oder Rhodium,auf Alumina, auf Silika, auf Bariumcarbonat, auf Bariumsulfat, auf Calciumcarbonat, auf Strontiumcarbonat, auf Kohle, auf Aktivkohle; Platin-Palladium-Gold-, Aluminum-Nickel-, Eisen-Nickel-, Lanthanoid-Nickel, Zirconium-Nickel-, Platin-Iridium-, Platin-Rhodium-Legierung; Raney^{®}-Nickel, Nickel-Zink-Eisen-Oxid; Palladium(II)-, Nickel(II)- ,Platin(II)-, Rhodiumchlorid, -bromid, -iodid, -fluorid, -hydrid, -oxid, -peroxid, -cyanid, -sulfat, -nitrat, -phosphid, -borid, -chromoxid, -cobaltoxid, -carbonathydroxid, -cyclohexanbutyrat, -hydroxid, -molybdat, -octanoat, -oxalat, -perchlorat, -phthalocyanin, -5,9,14,18,23,27,32,36-octabutoxy-2,3-naphthalocyanin, -sulfamat, -perchlorat, -thiocyanat, -bis(2,2,6,6-tetramethyl-3,5-heptanedionat), -propionat, -acetat, -stearat, -2-ethylhexanoat, -acetylacetonat, -hexafluoroacetylacetonat, -tetrafluoroborat, -thiosulfat, -trifluoroacetat, -phthalocyanintetrasulfonsäure Tetranatriumsalz, -methyl, -cyclopentadienyl, -methylcyclopentadienyl, -ethylcyclopentadienyl, -pentamethylcyclopentadienyl, -2,3,7,8,12,13,17,18-octaethyl-21H,23H-porphin, -5,10,15,20-tetraphenyl-21H,23H-porphin, -bis(5-[[4-(dimethylamino)phenyl]imino]-8(5H)-quinolinon), -2,11,20,29-tetra-tert-butyl-2,3-naphthalocyanin, -2,9,16,23-tetraphenoxy-29H,31H-phthalocyanin, -5,10,15,20-tetrakis(pentafluorophenyl)-21H,23H-porphin und deren 1,4-Bis(diphenylphosphin)butan-, 1,3-Bis(diphenylphosphino)-propan-, 2-(2'-Di-tert-butylphosphin)biphenyl-, Acetonitril-, Benzonitril-, Ethylendiamin-, Chloroform-, 1,2-Bis(phenylsulfinyl)ethan-, 1,3-Bis(2,6-diisopropylphenyl)-imidazoliden)(3-chloropyridyl)-, 2'-(Dimethylamino)-2-biphenylyl-, Dinorbornylphosphin-, 2-(Dimethylamino-methyl)ferrocen-, Allyl-, Bis(Diphenylphosphino)butan-, (N-succinimidyl)bis-(triphenylphosphin)-, Dimethylphenylphosphin-, Methyldiphenylphosphin-, 1,10-Phenanthrolin-, 1,5-Cyclooctadien-, N,N,N',N'-Tetramethylethylendiamin-, Triphenylphosphin-, Tri-o-tolylphosphin-, Tricyclohexylphosphin-, Tributylphosphin-, Triethylphosphin-, 2,2'-Bis(diphenylphosphino)-1,1'-binaphthyl-, 1,3-Bis(2,6-düsopropylphenyl)imidazol-2-yliden-, 1,3-Bis(mesityl)imidazol-2-yliden-, 1,1'-Bis(di-phenylphosphino)ferrocen-, 1.2-Bis(dipheny)phosphino)ethan-, N-Methylimidazol-, 2,2'-Bipyridin-, (Bicyclo[2.2.1]-hepta-2,5-dien)-, Bis(di-tert-butyl(4-dimethylaminophenyl)-phosphin)-, Bis(tert.-butylisocyanid)-, 2-Methoxyethylether-, Ethylenglycoldimethylether-1,2-Dimethoxyethan-, Bis(1,3-diamino-2-propanol)-, Bis(N,N-diethylethylendiamin)-, 1,2-Diaminocyclohexan-, Pyridin-, 2,2':6',2"-terpyridin-, Diethylsulfid-, Ethylen-,Amin-Komplexe; Kalium-, Natrium-, Ammoniumhexachloropalladat(IV), Kalium-, Natrium-, Ammonium-tetrachloropalladat(II), Bromo(tri-tert-butylphosphin)palladium(I) Dimer, (2-Methyl-allyl)palladium(II)chlorid Dimer, Bis(dibenzylidenaceton)palladium(0), Tris(di-benzylidenaceton)dipalladium(0), Tetrakis(triphenylphosphin)palladium(0), Tetrakis-(tricyclohexylphosphin)palladium (0), Bis[1,2-bis(diphenylphosphin)ethan]-palladium(0), Bis(3,5,3',5'-dimethoxydibenzylidenaceton)palladium(0), Bis(tri-tert-butylphosphin)palladium(0), meso-Tetraphenyltetrabenzoporphin Palladium, Tetrakis(methyldiphenylphosphin)palladium(0), Tris(3,3',3"-phophinidyn-tris(benzolsulfonato)palladium(0) Nonanatriumsalz, 1,3-Bis(2,4,6-trimethylphenyl)-imidazol-2-yliden(1,4-naphthoquinon)palladium(0), 1,3-Bis(2,6-diisopropylphenyl)-imidazol-2-yliden(1,4-naphthoquinon)palladium(0), und deren Chloroform-Komplex;
Allylnickel(II)chlorid Dimer, Ammoniumnickel(II)sulfat,
Bis(1,5-cyclooctadien)nickel(0), Bis(triphenylphosphin)dicarbonylnickel(0), Tetrakis(triphenylphosphin)nickel(0), Tetrakis(triphenylphosphit)nickel(0), Kaliumhexafluoronickelat(IV), Kaliumtetra-cyanonickelat(II), Kaliumnickel(IV)paraperiodat, Dilithiumtetrabromonickelat(II), Kaliumtetracyanonickelat(II);
Platin(IV)chlorid, -oxid, -sulfid, Kalium-, Natrium-,
Ammoniumhexachloroplatinat(IV), Kalium-, Ammoniumtetrachloroplatinat(II), Kaliumtetracyanoplatinat(II), Trimethyl(methylcyclopentadienyl)platin(IV), cis-Diammintetrachloroplatin(IV), Kaliumtrichloro(ethylen)platinat(II), Natriumhexahydroxyplatinat(IV), Tetraaminplatin(II)tetrachloroplatinat(II), Tetrabutylammoniumhexachloroplatinat(IV), Ethylenbis(triphenylphosphin)platin(0), Platin(0)-1,3-divinyl-1,1,3,3-tetramethyldisiloxan, Platin(0)-2,4,6,8-tetramethyl-2,4,6,8-tetravinylcyclotetrasiloxan, Tetrakis-(triphenylphosphin)platin(0), Platinoctaethylporphyrin, Chloroplatinsäure, Carboplatin;
Chlorobis(ethylen)rhodium Dimer, Hexarhodiumhexadecacarbonyl, Chloro(1,5-cyclooctadien)rhodium Dimer, Chloro(norbomadien)-rhodium Dimer, Chloro(1,5-hexadien)rhodium Dimer.

Bevorzugt handelt es sich bei den Liganden um Phosphine der Formel (VII)

PR⁹₃ (VII)

in der die Reste R⁹unabhängig voneinander für Wasserstoff, geradkettiges, verzweigtes oder cyclisches C₁-C₂₀-Alkyl, C₁-C₂₀-Alkylaryl, C₂-C₂₀-Alkenyl, C₂-C₂₀-Alkinyl, C₁-C₂₀-Carboxyat, C₁-C₂₀-Alkoxy, C₁-C₂₀-Alkenyloxy, C₁-C₂₀-Alkinyloxy, C₂-C₂₀-Alkoxy-carbonyl, C₁-C₂₀-Alkylthio, C₁-C₂₀-Alkylsulfonyl, C₁-C₂₀-Alkylsulfinyl, Silyl und/oder deren Derivative und/oder durch wenigstens ein R¹⁰ substituiertes Phenyl- oder durch wenigstens ein R¹⁰ substituiertes Naphtyl stehen. R¹⁰ steht unabhängig voneinander für Wasserstoff, Fluor, Chlor, Brom, lod, NH₂, Nitro, Hydroxy, Cyano, Formyl, geradkettiges, verzweigtes oder cyclisches C₁-C₂₀-Alkyl, C₁-C₂₀-Alkoxy, HN(C₁-C₂₀-Alkyₗ), N(C₁-C₂₀-Alkyl)₂, -CO₂-(C₁-C₂₀-Alkyl), -CON(C₁-C₂₀-Alkyl)₂, -OCO(C₁-C₂₀-Alkyl), NHCO(C₁-C₂₀-Alkyl), C₁-C₂₀-Acyl, -SO₃M, -SO₂N(R¹¹)M, -CO₂M, -PO₃M₂, -AsO₃M₂, -SiO₂M, -C(CF₃)₂OM (M = H, Li, Na oder K), wobei R¹¹ Wasserstoff, Fluor, Chlor, Brom, Iod, geradkettiges, verzweigtes oder cyclisches C₁-C₂₀-Alkyl, C₂-C₂₀-Alkenyl, C₂-C₂₀-Alkinyl, C₁-C₂₀-Carboxyat, C₁-C₂₀-Alkoxy, C₁-C₂₀-Alkenyloxy, C₁-C₂₀-Alkinyloxy, C₂-C₂₀-Alkoxycarbonyl, C₁-C₂₀-Alkylthio, C₁-C₂₀-Alkylsulfonyl, C₁-C₂₀-Alkylsulfinyl, Silyl und/oder deren Derivative, Aryl, C₁-C₂₀-Arylalkyl, C₁-C₂₀-Alkylaryl, Phenyl und/oder Biphenyl bedeutet. Vorzugsweise sind alle Gruppen R⁹ identisch.

Geeignete Phosphine (VII) sind beispielsweise Trimethyl-, Triethyl-, Tripropyl-, Triisopropyl-, Tributyl-, Triisobutyl-, Triisopentyt-, Trihexyl-, Tricyclohexyl-, Trioctyl-, Tridecyl-, Triphenyl-, Diphenylmethyl-, Phenyldimethyl-, Tri(o-tolyl)-, Tri(p-tolyl)-, Ethyldiphenyl-, Dicyclohexylphenyl-, 2-Pyridyldiphenyl-, Bis(6-methyl-2pyridyl)-phenyl-, Tri-(p-chlorophenyl)-, Tri-(p-methoxyphenyl)-, Diphenyl(2-sulfonatophenyl)phosphin; Kalium-, Natrium- und Ammoniumsalze von Diphenyl(3-sulfonatophenyl)phosphin, Bis(4,6-dimethyl-3-sulfonatophenyl)(2,4-dimethylphenyl)phosphin, Bis(3-sulfonatophenyl)phenylphosphinen, Tris(4,6-dimethyl-3-sulfonatophenyl)phosphinen, Tris(2-sulfonatophenyl)phosphinen, Tris(3-sulfonatophenyl)phosphinen;
2-Bis(diphenylphosphinoethyl)trimethylammoniumiodid, 2'-Dicyclohexylphosphino-2,6-dimethoxy-3-sulfonato-1,1'-biphenyl Natriumsalz, Trimethylphosphit und/oder Triphenylphosphit.

Besonders bevorzugt handelt es sich bei den Liganden um bidentate Liganden der allgemeinen Formel

R⁹M"-Z-M" R⁹ (VIII).

In dieser Formel repräsentieren M" unabhängig voneinander N, P, As oder Sb. Bevorzugt sind die beiden M" gleich und besonders bevorzugt steht M" für ein Phosphoratom.

Jede Gruppe R⁹ repräsentiert unabhängig voneinander die unter Formel (VII) beschrieben Reste. Vorzugsweise sind alle Gruppen R⁹ identisch.

Z stellt bevorzugt eine bivalente Überbrückungsgruppe dar, die wenigstens 1 Brückenatom enthält, wobei bevorzugt 2 bis 6 Brückenatome enthalten sind.

Brückenatome können ausgewählt werden aus C-, N-, O-, Si- und S-Atomen. Bevorzugt ist Z eine organische Überbrückungsgruppe, die wenigstens ein Kohlenstoffatom enthält. Bevorzugt ist Z eine organische Überbrückungsgruppe, die 1 bis 6 Brückenatome enthält, wovon wenigstens zwei Kohlenstoffatome sind, die unsubstituiert oder substituiert sein können.

Bevorzugte Gruppen Z sind -CH₂-, -CH₂-CH₂-, -CH₂-CH₂-CH₂-,
-CH₂-CH(CH₃)-CH₂-, -CH₂-C(CH₃)₂-CH₂-, -CH₂-C(C₂H₅)-CH₂-, -CH₂-Si(CH₃)₂-CH₂-, -CH₂-O-CH₂-, -CH₂-CH₂-CH₂-CH₂-, -CH₂-CH(C₂H₅)-CH₂-, -CH₂-CH(n-Pr)-CH und -CH₂-CH(n-Bu)-CH₂-, unsubstituierte oder substituierte 1,2-Phenyl-, 1,2-Cyclohexyl-, 1,1'- oder 1,2-Ferrocenyl-Reste, 2,2'-(1,1'-Biphenyl)-, 4,5-Xanthen- und/oder Oxydi-2,1-phenylen-Reste.

Geeignete bidentate Phosphinliganden (VIII) sind beispielsweise 1,2-Bis(dimethyl-), 1,2-Bis(diethyl-), 1,2-Bis(dipropyl-), 1,2-Bis(diisopropyl-), 1,2-Bis(dibutyl-), 1,2-Bis(di-tert.-butyl-), 1,2-Bis(dicyclohexyl-) und 1,2-Bis(diphenylphosphino)ethan; 1,3-Bis(dicyclohexyl-), 1,3-Bis(düsopropyl-), 1,3-Bis(di-tert.-butyl-) und 1,3-Bis(diphenylphosphino)propan; 1,4-Bis-(diisopropyl-) und 1,4-Bis(diphenylphosphino)butan; 1,5-Bis(dicyclohexylphosphino)pentan; 1,2-Bis(di-tert.-butyl-), 1,2-Bis(di-phenyl-), 1,2-Bis(di-cyclohexyl-), 1,2-Bis(dicyclo-pentyl-), 1,3-Bis(di-tert.-butyl-), 1,3-Bis(diphenyl-), 1,3 Bis(di-cyclohexyl-) und 1,3-Bis(dicyclopentylphosphino)benzol; 9,9-Dimethyl-4,5-bis(diphenylphosphino)xanthen, 9,9-Dimethyl-4,5-bis(diphenylphosphino)-2,7-di-tert.-butylxanthen, 9,9-Dimethyl-4,5-bis(di-tert.-butylphosphino)xanthen, 1,1'-Bis(diphenylphosphino)-ferrocen, 2,2'-Bis(diphenylphosphino)-1,1'-binaphthyl, 2,2'-Bis(di-p-tolylphosphino)-1,1'-binaphthyl, (Oxydi-2,1-phenylen)bis(diphenylphosphin), 2,5-(Di-isopropylphos-pholano)benzol, 2,3-O-Isopropropyliden-2,3-dihydroxy-1,4-bis(diphenylphosphino)-butan, 2,2'-Bis(di-tert.-butylphosphino)-1,1'-biphenyl, 2,2'-Bis(dicyclohexylphosphino)-1,1'-biphenyl, 2,2'-Bis(diphenylphosphino)-1,1'-biphenyl, 2-(Di-tert.-butylphosphino)-2'-(N,N-dimethylamino)biphenyl, 2-(Dicyclohexylphosphino)-2'-(N,N-dimethylamino)-biphenyl, 2-(Diphenylphosphino)-2'-(N,N-dimethylamino)biphenyl, 2-(Diphenylphos-phino)ethylamin, 2-[2-(Diphenylphosphino)ethyl]pyridin; Kalium-, Natrium- und Ammoniumsalze von 1,2-Bis(di-4-sulfonatophenylphosphino)-benzol, (2,2'-Bis [[bis(3-sulfonato-phenyl)phosphino]methyl]-4,4',7,7'-tetrasulfonato-1,1'-binapthyl, (2,2'-Bis[[bis(3-sulfonatophenyl)phosphino]methyl]-5,5'-tetrasulfonato-1,1'-biphenyl, (2,2'-Bis [[bis(3-sulfonatophenyl)phosphino]methyl]-1,1'-binapthyl, (2,2'-Bis[[bis(3-sulfonatophenyl)phosphino]-methyl]-1,1'-biphenyl, 9,9-Dimethyl-4,5-bis(diphenyl-phosphino)-2,7-sulfonatoxanthen, 9,9-Dimethyl-4,5-bis(di-tert.-butylphosphino)-2,7-sulfonatoxanthen, 1,2-Bis(di-4-sulfonatophenylphosphino}-benzol, Meso-tetrakis(4-sulfonatophenyl)porphin, Meso-tetrakis(2,6-dichloro-3-sulfonatophenyl)porphin, Meso-tetrakis(3-sulfonatomesityl)porphin, Tetrakis(4-carboxyphenyl)porphin und 5,11,17,23-Sulfonato-25,26,27,28-tetrahydroxycalix[4]aren.

Zudem können die Liganden der Formel (VII) und (VIII) durch die Reste R⁹ und/oder die Überbrückungsgruppe an ein geeignetes Polymer oder anorganisches Substrat gebunden sein.

Das Katalysatorsystem hat ein Übergangsmetall-Ligand-Molverhältnis von 1:0,01 bis 1:100, bevorzugt von 1:0,05 bis 1:10 und insbesondere von 1:1 bis 1:4.

Bevorzugt erfolgen die Umsetzungen in den Verfahrensstufen a), b), c) und d) wahlweise in einer Atmosphäre, die weitere gasförmige Bestandteile wie zum Beispiel Stickstoff, Sauerstoff, Argon, Kohlendioxid enthält; die Temperatur beträgt -20 bis 340 °C, insbesondere 20 bis 180 °C und der Gesamtdruck von 1 bis 100 bar.

Die lsolierung der Produkte und/oder des Übergangsmetalls und/oder der Übergangsmetallverbindung und/oder Katalysatorsystems und/oder des Liganden und/oder der Edukte nach den Verfahrensstufen a), b), c) und d) erfolgt wahlweise durch Destillation oder Rektifikation, durch Kristallisation oder Fällen, durch Filtration oder Zentrifugieren, durch Adsorption oder Chromatographie oder anderen bekannten Methoden.

Erfindungsgemäß werden Lösungsmittel, Hilfsmittel und ggf. andere flüchtige Bestandteile durch z. B. Destillation, Filtration und/oder Extraktion abgetrennt.

Bevorzugt erfolgt die Umsetzungen in den Verfahrensstufen a), b), c) und d) wahlweise in Absorptionskolonnen, Sprühtürmen, Blasensäulen, Rührkesseln, Reiselbettreaktor, Strömumgsrohren, Schlaufenreaktoren und/oder Knetern.

Geeignete Mischorgane sind z. B. Anker-, Blatt-, MIG-, Propeller-, Impeller-, Turbinen-, Kreuz-Rührer, Dispergierscheiben, Hohl-(Begasungs-)-Rührer, Rotor-Stator-Mischer, statische Mischer, Venturi-Düsen und/oder Mammutpumpen.

Bevorzugt erfahren die Reaktionslösungen/-mischungen dabei eine Mischintensität, die einer Rotations-Reynolds-Zahl von 1 bis 1000000, bevorzugt von 100 bis 100000 entspricht.

Bevorzugt erfolgt eine intensive Durchmischung der jeweiligen Reaktionspartner etc. unter einem Energieeintrag von 0,080 bis 10 kW/m³, bevorzugt 0,30 - 1,65 kW/m³.

Bevorzugt wirkt der jeweilige Katalysator A oder C während der Umsetzung homogen und/oder heterogen. Daher wirkt der jeweils heterogen wirkende Katalysator während der Umsetzung als Suspension oder an eine feste Phase gebunden.

Bevorzugt wird der Katalysator A vor der Umsetzung und/oder zu Beginn der Umsetzung und/oder während der Umsetzung in situ generiert.

Bevorzugt erfolgt die jeweilige Umsetzung in einem Lösungsmittel als Ein-Phasen-System in homogener oder heterogener Mischung und/oder in der Gasphase.

Wird ein Mehr-Phasen-System verwendet kann zusätzlich ein Phasentransferkatalysor eingesetzt werden.

Die erfindungsgemäßen Reaktionen können in flüssiger Phase, in der Gasphase oder auch in überkritischer Phase durchgeführt werden. Dabei wird der jeweilige Katalysator A oder C bei Flüssigkeiten vorzugsweise homogen oder als Suspension eingesetzt, während bei Gasphasen- oder überkritischer Fahrweise eine Festbettanordnung von Vorteil ist.

Geeignete Lösungsmittel sind Wasser, Alkohole wie z.B. Methanol, Ethanol, i-Propanol, n-Propanol, n-Butanol, i-Butanol, t-Butanol, n-Amylalkohol, i-Amylalkohol, t-Amylalkohol, n-Hexanol, n-Octanol, i-Octanol, n-Tridecanol, Benzylalkohol etc. Bevorzugt sind weiterhin Glycole wie z.B. Ethylenglycol, 1,2-Propandiol, 1,3-Propandiol, 1,3-Butandiol, 1,4-Butandiol, Diethylenglycol etc.; aliphatische Kohlenwasserstoffe wie Pentan, Hexan, Heptan, Octan, und Petrolether, Petroleumbenzin, Kerosin, Petroleum, Paraffinöl etc.; aromatisch Kohlenwasserstoffe wie Benzol, Toluol, Xylol, Mesitylen, Ethylbenzol, Diethylbenzol etc.; Halogenkohlenwasserstoffe wie Methylenchlorid, Chloroform, 1,2-Dichloroethan, Chlorobenzol, Tetrachlorkohlenstoff, Tetrabromoethylen etc.; alicyclische Kohlenwasserstoffe wie Cyclopentan, Cyclohexan und Methylcyclohexan etc.; Ether wie Anisol (Methylphenylether), t-Butylmethylether, Dibenzylether, Diethylether, Dioxan, Diphenylether, Methylvinylether, Tetrahydrofuran, Triisopropylether etc.; Glycolether wie Diethylenglycoldiethylether, Diethylenglycoldimethylether (Diglyme), Diethylenglycolmonobutylether, Diethylenglycolmonomethylether, 1,2-Dimethoxyethan (DME Monoglyme), Ethylenglycolmonobutylether, Triethylenglycoldimethylether (Triglyme), Triethylenglycolmonomethylether etc.; Ketone wie Aceton, Diisobutylketon, Methyl-n-propylketon; Methylethylketon, Methyl-i-butylketon etc; Ester wie Methylformat, Methylacetat, Ethylacetat, n-Propylacetat und n-Butylacetat etc.; Carbonsäuren wie Ameisensäure, Essigsäure, Propionsäure, Buttersäure etc.; einzeln oder in Kombination miteinander.

Geeignete Lösungsmittel sind auch die eingesetzten Olefine und Phosphinsäurequellen. Diese bieten Vorteile in Form einer höheren Raum-ZeitAusbeute.

Bevorzugt wird die Umsetzung unter dem eigenen Dampfdruck des Olefins und/oder des Lösungsmittels durchgeführt.

Bevorzugt sind R¹, R², R³, R⁴ des Olefins (IV) gleich oder verschieden und bedeuten, unabhängig voneinander, H, Methyl, Ethyl, n-Propyl, Isopropyl, n-Butyl, Isobutyl, tert.-Butyl und/oder Phenyl.

Bevorzugt werden auch funktionalisierte Olefine wie Allylisothiocyanat, Allylmethacrylat, 2-Allylphenol, N-Allylthioharnstoff, 2-(Allylthio)-2-thiazolin, Allyltrimethylsillan, Allylacetat, Allylacetoacetat, Allylalkohol, Allylamin, Allylbenzol, Allylcyanid, Allyl-(cyanacetat), Allylanisol, trans-2-Pentenal, cis-2-Pentennitril, 1-Penten-3-ol, 4-Penten-1-ol, 4-Penten-2-ol, trans-2-Hexenal, trans-2-Hexen-1-ol, cis-3-Hexen-1-ol, 5-Hexen-1-ol, Styrol, -Methylstyrol, 4-Methylstyrol, Vinylacetat, 9-Vinylanthracen, 2-Vinylpyridin, 4-Vinylpyridin und 1-Vinyl-2-pyrrolidon eingesetzt.

Bevorzugt erfolgt die Umsetzung bei einem Partialdruck des Olefins von 0,01 - 100 bar, besonders bevorzugt bei einem Partialdruck des Olefins von 0,1 -10 bar.

Bevorzugt erfolgt die Umsetzung in einem Phosphinsäure-Olefin-Molverhältnis von 1:10000 bis 1:0,001, besonders bevorzugt im Verhältnis von 1:30 bis 1:0,01.

Bevorzugt erfolgt die Umsetzung in einem Phosphinsäure-Katalysator-Molverhältnis von 1:1 bis 1:0,00000001, besonders bevorzugt bei 1:0,01 bis 1:0,000001.

Bevorzugt erfolgt die Umsetzung in einem Phosphinsäure-Lösungsmittel-Molverhältnis von 1:10000 bis 1:0, besonders bevorzugt bei 1:50 bis 1:1.

Ein erfindungsgemäßes Verfahren zur Herstellung von Verbindungen der Formel (II) ist dadurch gekennzeichnet, dass man eine Phosphinsäurequelle mit Olefinen in Gegenwart eines Katalysators umsetzt und das Produkt (II) (Alkylphosphonigsäure bzw. -salze, -ester) von Katalysator, Übergangsmetall bzw. Übergangsmetallverbindung, Ligand, Komplexbildner, Salzen und Nebenprodukten befreit wird.

Erfindungsgemäß wird der Katalysator, das Katalysatorsystem, das Übergangsmetall und/oder die Übergangsmetallverbindung abgetrennt durch Zugabe eines Hilfsmittels 1 und Entfernen des Katalysators, des Katalysatorsystems, des Übergangsmetalls und/oder der Übergangsmetallverbindung durch Extraktion und/oder Filtration.

Erfindungsgemäß wird der Ligand und/oder Komplexbildner durch Extraktion mit Hilfsmittel 2 und/oder Destillation mit Hilfsmittel 2 abgetrennt.

Hilfsmittel 1 ist bevorzugt Wasser und/oder mindestens ein Vertreter der Familie der Metallfänger (Metal Scavenger). Bevorzugte Metallfänger sind Metalloxide wie etwa Aluminiumoxid, Siliciumdioxid, Titandioxid, Zirkoniumdioxid, Zinkoxid, Nickeloxid, Vanadiumoxid, Chromoxid, Magnesiumoxid, Celite^{®}, Kieselgur; Metallcarbonate wie etwa Bariumcarbonat, Calciumcarbonat, Strontiumcarbonat; Metallsulfate wie etwa Bariumsulfat, Calciumsulfat, Strontiumsulfat; Metallphosphate wie etwa Aluminiumphosphat, VanadiumphosphatM Metallcarbide wie etwa Siliconcarbid; Metallaluminate wie etwa Calciumaluminat; Metallsilikate wie etwa Aluminiumsilikat, Kreiden, Zeolithe, Bentonit, Montmorillonit, Hectorit; funktionalisierte Silikate, funktionalisierte Silikagele wie etwa SiliaBond^{®}, QuadraSil™; funktionalisierte Polysiloxane wie etwa Deloxan^{®}; Metallnitride, Kohle, Aktivkohle, Mullite, Bauxite, Antimonite, Scheelite, Perovskite, Hydrotalcite, funktionalisierte und unfunktionalisierte Cellulose, Chitosan, Keratin, Heteropolyanionen, Ionentauscher wie etwa Amberlite^{™}, Amberjet^{™}, Ambersep^{™}, Dowex^{®}, Lewatit^{®}, ScavNet^{®}; funktionalisierte Polymere wie etwa Chelex^{®}, QuadraPure^{™}, Smopex^{®}, PolyOrgs^{®}; polymergebundene Phosphane, Phosphanoxide, Phosphinate, Phosphonate, Phosphate, Amine, Ammoniumsalze, Amide, Thioamide, Harnstoffe, Thioharnstoffe, Triazine, Imidazole, Pyrazole, Pyridine, Pyrimidine, Pyrazine, Thiole, Thiolether, Thiolester, Alkohole, Alkoxide, Ether, Ester, Carbonsäuren, Acetate, Acetale, Peptide, Hetarene, Polyethylenimin/Siliciumdioxid und/oder Dendrimere.

Bevorzugt wird Hilfsmittel 1 in Mengen zugesetzt, die einer 0,1 - 40 gew.-%igen Beladung des Metalls auf dem Hilfsmittel 1 entsprechen.

Bevorzugt wird Hilfsmittel 1 bei Temperaturen von 20 - 90°C eingesetzt.

Bevorzugt beträgt die Verweilzeit von Hilfsmittel 1 0,5 - 360 Minuten.

Hilfsmittel 2 ist bevorzugt das vorgenannte, erfindungsgemäße Lösungsmittel, wie sie bevorzugt in der Verfahrensstufe a) eingesetzt werden.

Die Veresterung der mono-carboxyfunktionalisierten Dialkylphosphinsäure (III) bzw. der mono-funktionalisierten Dialkylphosphinsäure (VI) und/oder (VI') bzw. der Alkylphosphonigsäuredrivate (II) sowie der Phosphinsäurequelle (I) zu den entsprechenden Estern kann beispielsweise durch Umsetzung mit höhersiedenden Alkoholen unter Entfernung des gebildeten Wassers durch Azeotropdestillation oder durch Umsetzung mit Epoxiden (Alkylenoxiden) erreicht werden.

Bevorzugt wird hierbei nach Schritt a) die Alkylphosphonigsäure (II) mit einem Alkohol der allgemeinen Formel M-OH und/oder M'-OH oder durch Umsetzung mit Alkylenoxiden, wie nachfolgend angeführt, direkt verestert.

Bevorzugt sind M-OH primäre, sekundäre oder tertiäre Alkohole mit einer Kohlen-stoffkettenlänge von C₁-C₁₈. Besonders bevorzugt sind Methanol, Ethanol, Propanol, Isopropanol, n-Butanol, 2-Butanol, tert.-Butanol, Amylalkohol und/oder Hexanol.

Bevorzugt sind M'-OH Ethylenglykol, 1,2-Propylenglykol, 1,3-Propylenglykol, 1,4-Butandiol, 2,2-Dimethylpropan-1,3-diol, Neopentylglykol, 1,6-Hexandiol, 1,4-Cyclohexan-dimethanol, Glycerin, Trishydroxymethylethan, Trishydroxymethylpropan, Pentaerythrit, Sorbit, Mannit, α-Naphthol, Polyethylenglykole, Polypropylenglykole und/oder EO-PO-Blockpolymere.

Geeignet sind als M-OH und M'-OH auch ein- oder mehrwertige, ungesättigte Alkohole mit einer Kohlenstoffkettenlänge von C₁-C₁₈, etwa n-Buten-2-ol-1, 1,4-Butendiol und Allylalkohol.

Geeignet sind als M-OH und M'-OH auch Umsetzungsprodukte von einwertigen Alkoholen mit einem oder mehreren Molekülen von Alkylenoxiden, bevorzugt mit Ethylenoxid und/oder 1,2-Propylenoxid. Bevorzugt sind 2-Methoxyethanol, 2-Ethoxyethanol, 2-n-Butoxy-ethanol, 2-(2'-Ethyl-hexyloxy)-ethanol, 2-n-Dodecoxy-ethanol, Methyldiglykol, Ethyldiglykol, Isopropyldiglykol, Fettalkoholpolyglykolether und Arylpolyglykolether.

Bevorzugt sind M-OH und M'-OH auch Umsetzungsprodukte von mehrwertigen Alkoholen mit einem oder mehreren Molekülen Alkylenoxid, insbesondere Diglykol und Triglykol sowie Addukte von 1 bis 6 Molekülen Ethylenoxid oder Propylenoxid an Glycerin, Trishydroxymethylpropan oder Pentaerythrit.

Als M-OH und M'-OH können auch Umsetzungsprodukte von Wasser mit einem oder mehreren Molekülen Alkylenoxid eingesetzt werden. Bevorzugt sind Polyethylenglykole und Poly-1,2-propylenglykole verschiedener Molekulargrößen mit einem mittleren Molgewicht von 100-1000 g/mol, besonders bevorzugt von 150-350 g/mol.

Bevorzugt sind als M-OH und M'-OH auch Umsetzungsprodukte von Ethylenoxid mit Poly-1,2-propylenglykolen oder Fettalkoholpropylenglykole; ebenso Umsetzungsprodukte von 1,2-Propylenoxid mit Polyethylenglykolen oder Fettalkoholethoxylaten. Bevorzugt sind solche Umsetzungsprodukte mit einem mittleren Molgewicht von 100-1000 g/mol, besonders bevorzugt von 150-450 g/mol.

Einsetzbar sind als M-OH und M'-OH auch Umsetzungsprodukte von Alkylenoxiden mit Ammoniak, primären oder sekundären Aminen, Schwefelwasserstoff, Merkaptanen, Sauerstoffsäuren des Phosphors und C₂-C₆-Dicarbonsäuren. Geeignete Umsetzungsprodukte von Ethylenoxid mit Stickstoffverbindungen sind Triethanolamin, Methyldiethanolamin, n-Butyl-diethanolamin, n-Dodecyl-diethanolamin, Dimethylethanolamin, n-Butyl-methyl-ethanolamin, Di-n-butyl-ethanolamin, n-Dodecylmethyl-ethanolamin, Tetrahydroxyethyl-ethylendiamin oder Pentahydroxyethyl-diethylentriamin.

Bevorzugte Alkylenoxide sind Ethylenoxid, 1,2-Propylenoxid, 1,2-Epoxybutan, 1,2-poxyethylbenzol, (2,3-Epoxypropyl)benzol, 2,3-Epoxy-1-propanol und 3,4-Epoxy-1-buten.

Geeignete Lösungsmittel sind die in Verfahrensschritt a) genannten Lösungsmittel und auch die eingesetzten Alkohole M-OH, M'-OH und die Alkylenoxide. Diese bieten Vorteile in Form einer höheren Raum-Zeit-Ausbeute.

Bevorzugt wird die Umsetzung unter dem eigenen Dampfdruck des eingesetzten Alkohols M-OH, M'-OH und Alkylenoxids und/oder des Lösungsmittels durchgeführt.

Bevorzugt erfolgt die Umsetzung bei einem Partialdruck des eingesetzten Alkohols M-H, M'-OH und Alkylenoxids von 0,1 - 100 bar, besonders bevorzugt bei einem Partialdruck des Alkohols von 0, -10 bar.

Bevorzugt wird die Umsetzung bei einer Temperatur von -20 bis 340 °C durchgeführt, besonders bevorzugt bei einer Temperatur von 20 bis 180 °C.

Bevorzugt erfolgt die Umsetzung bei einem Gesamtdruck von 1 bis 100 bar.

Bevorzugt erfolgt die Umsetzung in einem Molverhältnis der Alkohol- bzw. Alkylenoxidkomponente zu der Phosphinsäurequelle (I) bzw. Alkylphosphonigsäure (II) bzw. mono-funktionalisierten Dialkylphosphinsäure (VI) und/oder VI' bzw. mono-carboxyfunktionalisierte Dialkylphosphinsäure (III) von 10000:1 bis 0,01:1, besonders bevorzugt im Verhältnis von 1000:1 bis 0,1:1.

Bevorzugt erfolgt die Umsetzung in einem Molverhältnis der Phosphinsäurequelle (I) bzw. Alkylphosphonigsäure (II) bzw. mono-funktionalisierten Dialkylphosphinsäure (VI) und/oder VI' bzw. mono-carboxyfunktionalisierte Dialkylphosphinsäure (III) zum Lösungsmittel von 1:10000 bis 1:0, besonders bevorzugt in einem Phosphinsäure-Lösungsmittel-Molverhältnis von 1:50 bis 1:1.

Besonders bevorzugte Katalysatoren B, wie sie in der Verfahrensstufe b) eingesetzt werden, sind Peroxo-Verbindungen wie Peroxo-monoschwefelsäure, Kaliummonopersulfat (Kaliumperoxomonosulfat), Caroat^{(™)}, Oxone^{(™)}, Peroxodischwefelsäure, Kaliumpersulfat (Kaliumperoxodisulfat), Natriumpersulfat (Natriumperoxodisulfat), Ammoniumpersulfat (Ammoniumperoxodisulfat).

Besonders bevorzugte Katalysatoren B sind Verbindungen, die im Lösemittelsystem Peroxide bilden können wie Natriumperoxid, -hydrate, Natriumperoxiddiperoxohydrat, -hydrate, Lithiumperoxid, -hydrate, Calciumperoxid, Strontiumperoxid, Bariumperoxid, Magnesiumperoxid, Zinkperoxid, Kaliumhyperoxid, -hydrate, Natriumperoxoborat, -hydrate, Kaliumperoxoboratperoxohydrat, Magnesiumperoxoborat, Calciumperoxoborat, Bariumperoxoborat, Strontiumperoxoborat, Kaliumperoxoborat, Peroxomonophosphorsäure, Peroxodiphosphorsäure, Kaliumperoxodiphosphat, Ammoniumperoxodiphosphat, Kaliumammoniumperoxodiphosphate, Natriumcarbonatperoxohydrat, Harnstoffperoxohydrat, Ammoniumoxalatperoxid, Bariumperoxidperoxohydrat, Bariumperoxidperoxohydrat, Calciumhydrogenperoxide, Calciumperoxidperoxohydrat, Ammoniumtriphosphatdiperoxophosphathydrat, Kaliumfluoridperoxohydrat, Kaliumfluoridtriperoxohydrat, Kaliumfluoriddiperoxohydrat, Natriumpyrophosphatdiperoxohydrat, Natriumpyrophosphatdiperoxohydratoctahydrat, Kaliumacetatperoxohydrat, Natriumphosphatperoxohydrat, Natriumsilicatperoxohydrat.

Bevorzugte Katalysatoren B sind Wasserstoffperoxid, Perameisensäure, Peressigsäure, Benzoylperoxid, Di-tert-butylperoxid, Dicumylperoxid, 2,4-Dichlorobenzoylperoxid, Decanoylperoxid, Laurylperoxid, Cumolhydroperoxid, Pinenhydroperoxid, p-Menthanhydroperoxid, tert-Butylhydroperoxid, Acetylacetonperoxid, Methylethylketonperoxid, Bemsteinsäureperoxid, Dicetylperoxydicarbonat, tert-Butylperoxyacetat, tert-Butylperoxymaleinsäure, tert-Butylperoxybenzoat, Acetylcyclohexylsulfonylperoxid.

Bevorzugte Katalysatoren B sind wasserlösliche Azo-Verbindungen. Besonders bevorzugt sind Azoinitiatoren wie VAZO^{®} 52 2,2'-Azobis(2,4-dimethyl-valeronitril), VAZO^{®} 64 (Azo-bis-(isobutyronitril), AIBN), VAZO^{®} 67 2,2'-Azobis(2-methylbutyronitril), VAZO^{®} 88 1,1'-Azobis(cyclohexane-1-carbonitril), VAZO^{®} 68 der Fa. Dupont-Biesteritz, V-70 2,2'-Azobis(4-methoxy-2,4-dimethyl valeronitril), V-65 2,2'-Azobis(2,4-dimethyl-valeronitril), V-601 Dimethyl 2,2'-azobis(2-methylpropionat), V-59 2,2'-Azobis(2-methylbutyronitril), V-40 1,1'-Azobis(cyclohexane-1-carbonitril), VF-096 2,2'-Azobis[N-(2-propenyl)-2-methylpropionamid], V-30 1-[(cyano-1-methylethyl)azo]formamid, VAm-110 2,2'-Azobis(N-butyl-2-methyl-propionamid), VAm-111 2,2'-Azobis(N-cyclohexyl-2-methylpropionamid), VA-046B 2,2'-Azobis[2-(2-imidazolin-2-yl)propandisulfatedi-hydrate, VA-057 2,2'-Azobis[N-(2-carboxyethyl)-2-methylpropionamidin]tetrahydrat, VA-061 2,2'-Azobis[2-(2-imidazolin-2-yl)propan], VA-080 2,2'-Azobis{2-methyl-N-[1,1-bis(hydroxymethyl)-2-hydroxyethyl] propionamid, VA-085 2,2'-Azobis{2-methyl-N-[2-(1-hydroxybuthyl)]propionamid}, VA-086 2,2'-Azobis[2-methyl-N-(2-hydroxyethyl)-propionamid] von Wako Chemicals.

Geeignet sind auch Azoinitiatoren wie 2-tert-Butylazo-2-cyanopropan, Dimethylazodiisobutyrat, Azodiisobutyronitril, 2-tert-Butylazo-1-cyanocyclohexan, 1-tert-Amylazo-1-cyanocyclohexan. Weiterhin sind bevorzugt Alkylperketale wie 2,2-Bis-(tert-butylperoxy)butan, Ethyl-3,3-bis(tert-butylperoxy)butyrat, 1,1-Di-(tert-butylperoxy)cyclohexan.

Bevorzugte Katalysatoren B sind auch Metalle, Metallhydride und Metallalkoholate wie zum Beispiel Lithium, Lithiumhydrid, Lithiumaluminiumhydrid, Methyllithium, Butyllithium, tert.-Butyllithium, Lithiumdiisopropylamid, Natrium, Natriumhydrid, Natriumborhydrid, Natriummethanolat, Natriumethanolat oder Natriumbutylat, Kaliummethanolat, Kaliumethanolat oder Kaliumbutylat.

Bevorzugt wird der Katalysator B in Mengen von 0,05 bis 5 mol-% bezüglich der jeweiligen Allylalkohole (V) und/oder Acroleine (V') eingesetzt.

Bevorzugt wird der Katalysator B in Mengen von 0,001 bis 10 mol-%, bezogen auf die phosphorhaltige Verbindung, eingesetzt.

Bevorzugt wird der Katalysator B mit einer Geschwindigkeit von 0,01 bis 10 mol-% Katalysator pro Stunde, bezogen auf die phosphorhaltige Verbindung, zudosiert.

Geeignete Lösungsmittel sind die, wie sie weiter vorne in Verfahrensstufe a) eingesetzt werden.

Bevorzugt wird der Iniator B mit einer Geschwindigkeit von 0,01 bis 10 mol-% Katalysator pro Stunde, bezogen auf die phosphorhaltige Verbindung, zudosiert.

Bevorzugt erfolgt die Umsetzung der Alkylphosphonigsäuren (II) mit einem Allylalkohol (V) und/oder Acrolein (V') bei einer Temperatur von 0 bis 250 °C, besonders bevorzugt bei 20 bis 200 °C und insbesondere bei 50 bis 150 °C.

Bevorzugt besteht die Atmosphäre bei der Umsetzung mit mit einem Allylalkohol (V) und/oder Acrolein (V') zu 50 bis 99,9 Gew.-% aus Bestandteilen des Lösungsmittels und Allylalkohol (V) und/oder Acrolein (V'), bevorzugt 70-95 Gew.-%.

Bevorzugt erfolgt die Umsetzung während des Zusatz von Allylalkohol (V) und/oder Acrolein (V') bei einem Druck von 1 -20 bar.

In einer weiteren Ausführungsform des Verfahrens wird das nach Verfahrensstufe
a) und/oder b) erhaltene Produktgemisch aufgearbeitet.

In einer weiteren Ausführungsform des Verfahrens wird das nach Verfahrensstufe
a) erhaltene Produktgemisch aufgearbeitet und danach die nach Verfahrensstufe
b) erhaltenen mono-funktionalisierten Dialkylphosphinsäuren und/oder deren Ester und Alkalisalze in Verfahrensstufe c) umgesetzt.

Gegenstand der Erfindung ist zudem ein Verfahren in Schritt b) zur kontinuierlichen Herstellung von mono-funktionalisierten
Dialkylphosphinsäureestern (VI) durch Umsetzung von
Alkylphosphonigsäureestern (II) mit einem Acrolein (VI') in Gegenwart von Metallalkoholaten (Katalysator B), welches dadurch gekennzeichnet ist, dass man
a) in einem zur Kreislaufführung des Reaktionsgemisches ausgebildeten, in sich geschlossenen und mit Kühleinrichtungen sowie Überlauf versehenen Reaktor eine dem Reaktorvolumen entsprechende Volumenmenge des herzustellenden mono-funktionalisierten Dialkylphosphinsäureestern (VI), gegebenenfalls im Gemisch mit dem Metallalkoholat entsprechenden Alkohol als Lösungsmittel, vorlegt und im Kreislauf führt; dass man
b) in den Reaktor kontinuierlich den Alkylphosphonigsäureester (II), das Acrolein (VI') sowie eine alkoholische Lösung des Metallalkoholats unter Kühlung des im Kreislauf geführten Reaktorinhaltes einleitet und bei einer Temperatur von etwa Null bis 80 °C im Verlauf von etwa 5 - 120 Minuten umsetzt, wobei das Molverhältnis des Alkylphosphonigsäureesters (II) zu dem Acrolein (VI') etwa 1 zu 0,9 - 2 und die Menge des Metallalkoholats, bezogen auf den Alkylphosphonigsäureester (II), etwa 0,1 bis 5 Mol% beträgt; und dass man
c) über den Überlauf des Reaktors kontinuierlich ein das Verfahrensprodukt enthaltendes Gemisch abzieht und aus dem Gemisch den mono-funktionalisierten Dialkylphosphinsäureester (VI) durch Destillation abtrennt.

Eine bevorzugte Ausführungsform des erfindungsgemäßen Verfahrens besteht darin, dass man die Umsetzung der Reaktionskomponenten bei einer Temperatur von 20 bis 50 °C durchführt. Die Beschickung des Reaktors mit den Reaktionskomponenten und der Katalysatorlösung kann beispielsweise derart erfolgen, dass man
a) den Alkylphosphonigsäureester (11), das Acrolein (V') sowie die alkoholische Lösung des Metallalkoholats getrennt in den Reaktor einleitet
b) ein Gemisch des Alkylphosphonigsäureesters (II) mit dem Acrolein (V'), getrennt von der alkoholischen Lösung des Metallalkoholats, in den Reaktor einleitet oder
c) ein Gemisch des Alkylphosphonigsäureesters (II) mit der alkoholischen Lösung des Metallalkoholats, getrennt von dem Acrolein (V'), in den Reaktor einleitet.

Weiterhin ist es zweckmäßig, wenn der als Lösungsmittel eingesetzte Alkohol und/oder die alkoholische Komponente des Metallalkoholats der alkoholischen Komponente des Alkylphosphonigsäureesters (II) entspricht.

Im Falle des Einsatzes von Alkylphosphonigsäureester (II) und der alkoholischen Metallalkoholatlösung mit unterschiedlichen alkoholischen Komponenten wird ein Mischprodukt als Verfahrensprodukt erhalten.

Schließlich bestehen bevorzugte Merkmale der Erfindung darin, dass das Mol-verhältnis von Alkylphosphonigsäureester (II) zu Acrolein (VI') 1 zu 1 -1,3, die Menge an Katalysator B bezogen auf den Alkylphosphonigsäureester (II) 1-5 mol-% und die Menge des als Lösungsmittel eingesetzten Alkohols 0,1 - 1000 Mol pro Mol Alkylphosphonigsäureester beträgt.

Gemäß der erfindungsgemäßen Verfahrensweise wird es ermöglicht, in technischem Maßstab kontinuierlich mono-funktionalisierten Dialkylphosphinsäureester (VI') in einer bisher nicht erreichten Ausbeute von etwa 90 % der Theorie herzustellen.

Die im Schritt c) beschriebene Umsetzung zur mono-carboxyfunktionalisierten Dialkylphosphinsäure, deren Salze und Ester wird durch selektive Oxidation der mono-funktionalisierten Dialkylphosphinsäure, deren Salze oder Ester (VI) und/oder (VI') durch ein Oxidationsmittel, ein Oxidationsmittel und Wasser oder durch Sauerstoff und Wasser in Gegenwart eines Katalysators C erreicht.

Bevorzugte Oxidationsmittel und/oder Sauerstoff-Bildner sind Kaliumpermanganat, Braunstein, Chromtrioxid, Kaliumdichromat, Pyridindichromat, Pyridinchlorchromat, Collins-Reagenz, Jones-Reagenz, Corey-Gilman-Ganem-Reagenz, (Dess-Martin-)Periodinan, o-Iodoxy-benzoesäure, Rutheniumtetroxid, Rutheniumdioxid, Tetra-n-propyl-perruthenat, Rutheniumtrichlorid/Natriumperiodat, Rutheniumdioxid/Natriumperiodat, Chlor, Hypochlorit, Persäuren, wie z. B. Wasserstoffperoxid, Perameisensäure und Peressigsäure, Nitroxylradikale, wie z. B. 2,2,6,6-Tetramethylpiperidin-N-oxid (TEMPO).

Zusätzlich zu den o. g. Oxidationsmitteln und/oder Sauerstoff-Bildnern sind auch alle die geeignet, wie sie in Verfahrensschritt b) als Katalysator B genannt sind.

Bevorzugt erfolgt die Umsetzung in einem Dialkylphosphinsäure-Oxidationsmittel-Molverhältnis von 1:10 bis 1:0,1, besonders bevorzugt in einem Dialkylphosphinsäure-Oxidationsmittel-Molverhältnis von 1:2 bis 1:0,25.

Der Katalysator C, wie er für den Verfahrensschritt c) für die Umsetzung des mono-funktionalisiertes Dialkylphosphinsäurederivat (VI) oder (VI') mit Sauerstoff und Wasser zum Endprodukt, dem mono-carboxyfunktionalisierten Dialkylphosphinsäurederivat (III) eingesetzt wird, kann bevorzugt der Katalysator A sein.

Zusätzlich handelt es sich bevorzugt bei den Übergangsmetallen für den Katalysator C um Elemente der ersten Nebengruppe, wie etwa Gold.

Zusätzlich zu den unter Katalysator A aufgelisteten Quellen von Übergangsmetallen und Übergangsmetallverbindungen können auch die folgenden Übergangsmetalle und Übergangsmetallverbindungen eingesetzt werden:
Gold, colloidales Gold, Ruthenium, Ruthenium auf Aktivkohle, auf Kohle, auf Alumina, Platin-Palladium-Gold-, Gold-Nickel-, Gold-Germanium-, Gold-Platin-, Gold-Palladium-, Gold-Beryllium-, Platin-Ruthenium-, Palladium-Ruthenium-Legierung, Gold(I)- und/oder Gold(III)-, Ruthenium(II)- und/oder Ruthenium(III)-und/oder Ruthenium(IV)chlorid, -bromid, -iodid, -oxid, -cyanid, -kaliumcyanid, - natriumcyanid -sulfid, -sulfat, -hydrid, -nitrosylchlorid, -nitrosylnitrat, -bathophenanthrolindisulfonat Natriumsalz, -thiosulfat, -perchlorat, -cyclopentadienyl, -ethylcyclopentadienyl, -pentamethylcyclopentadienyl, -indenyl, -2-methylallyl, -propionat, -acetat, -acetylacetonat, -hexafluoroacetylacetonat, -tetrafluoroborat, -kaliumthiocyanat, -natriumthiocyanat, -trifluoroacetat, -bis(trifluoromethansulfonyl)imidat, -hexafluoroantimonat, -2-pyridincarboxylat, und deren 1,4-Bis(diphenylphosphin)-butan-, 1,3-Bis(diphenylphosphino)propan-, 2-(2'- Di-tert-butylphosphin)biphenyl-, Acetonitril-, Benzonitril-, Dinorbornylphosphin-, 1,4-Bis(diphenylphosphino)butan-, Dimethylphenylphosphin-, Methyldiphenylphosphin-, Triphenylphosphin-, Tri-o-tolylphosphin-, Tricyclohexylphosphin-, Tributylphosphin-, Tri-tert-butylphosphin-, Trimethylphosphin-, Triethylphosphin-, 2,2'-Bis(diphenylphos-phino)-1,1'-Binaphthyl-, 1,3-Bis(mesityl)imidazol-2-yliden-, 1,1'-Bis(diphenyl-phosphino)ferrocen-, (1,1'-Biphenyl-2-yl)di-tert-butylphosphin-, 1,3-Bis(2,6-diiso-propyl-phenyl)imidazol-2-yliden-, 2-Dicyclohexyl(2',4',6'-trisopropylbiphenyl)-phosphin-, Dimethylsulfid-, Tris(2,4-di-tert-butylphenyl)phosphit-, Tris(para-trifluoromethyl-phenyl)phosphin-, Bis(diphenyl-phosphino)methan-, 1,2-Bis(diphenylphosphino)ethan-, N-Methylimidazol-, 1,10-Phenanthrolin-, 4,7-Diphenyl-1,10-phenanthrolin-, 1,5-Cyclooctadien-, 1,3,5-Cyclooctatrien-, Napthalen-, p-Cymen-, 3-Methyl-2-butenyliden-, Benzyliden-, Pyridin-, 2,3,7,8,12,13,17,18-Octaethyl-21H,23H-porphin-, 5,10,15,20-Tetraphenyl-21 H,23H-porphin-, N,N,N',N'-Tetramethylethylendiamin-, Tri-o-tolylphosphin-, 2,2'-Bis(diphenylphosphino)-1,1'-binaphthyl-, 1,1'-Bis(diphenyl-phosphino)ferrocen-, 2,2'-Bipyridin-, (Bicyclo[2.2.1]hepta-2,5-dien)-, Bis(di-tert-butyl(4-dimethylaminophenyl)phosphin)-, 2-(Di-tert-butylphosphino)ethylamin-, (2-(Diphenyl-phosphino)ethylamin-, 1,3-Bis(2,4,6-trimethylphenyl)-2-imidazolidinyliden-, 1,2-Di-aminocyclohexan-, Pyridin-, Carbonyl-, Ethylendiamin-, Amin-Komplex; Kaliumdicyanoaurat(I), Natriumtetrachloroaurat(III), Kaliumgold(III)chlorid, Natriumaurothiomalat, Tris(triphenylphosphingold)oxonium tetrafluoroborat, Wasserstofftetrabromoaurat(III); Ammoniumhexachlororuthenat(IV), Kaliumaquapentachlororuthenat(III), (1,5-Cyclooctadien)-(1,3,5-cyclooctatrien)ruthenium, Trirutheniumdodecacarbonyl, Grubbs Katalysator.

Bevorzugt beträgt der Anteil an Katalysator C bezogen auf die eingesetzte monofunktionalisierte Dialkylphosphinsäure (VI) und/oder (VI') 0,00001 bis 20 mol-%, besonders bevorzugt 0,0001 bis 10 mol-%.

Bevorzugt erfolgt die Umsetzung in einem Phosphinsäure-Lösungsmittel-Molverhältnis von 1:10000 bis 1:0, besonders bevorzugt in einem Phosphinsäure-Lösungsmittel-Molverhältnis von 1:50 bis 1:1.

Bevorzugt erfolgt die Oxidation bei Temperaturen von 30 bis 120°C und besonders bevorzugt bei 50 bis 90 °C.

Bevorzugt beträgt die Reaktionszeit 0,1 bis 20 Stunden.

Bevorzugt erfolgt die Umsetzung bei einem Gesamtdruck von 1 bis 100 bar.

Geeignete Lösungsmittel für Verfahrensstufe c) sind die, wie sie weiter vorne in Verfahrensstufe a) eingesetzt werden.

Bevorzugt erfolgt die Umsetzung bei einem Partialdruck des Sauerstoffs von 0,01 - 100 bar, besonders bevorzugt bei 0,1 - 10 bar

Die Oxidation kann in flüssiger Phase, in der Gasphase oder auch in überkritischer Phase durchgeführt werden. Dabei wird der Katalysator bei Flüssigkeiten vorzugsweise homogen oder als Suspension eingesetzt, während als bei Gasphasen- oder überkritischer Fahrweise eine Festbettanordnung von Vorteil ist.

Bevorzugt wird der pH der Reaktionslösung durch Zugabe von Alkali- und/oder Erdalkaliverbindungen in einem Bereich von pH 6 bis 12 gehalten, besonders bevorzugt in einem Bereich von pH 6 bis 9.

Bevorzugte Alkali- und/oder Erdalkalimetalle sind Lithium, Natrium, Kalium, Magnesium, Calcium, Barium. Besonders bevorzugt sind Natrium, Kalium, Calcium, Barium.

Bevorzugte Verbindungen der Alkali- und Erdalkalimetalle sind deren Oxide, Hydroxide, Carbonate und Carboxylate.

Bevorzugte Alkali- und/oder Erdalkalimetallverbindungen sind Lithium, Lithiumhydroxid, Lithiumhydrid, Natrium, Natriumhydroxid, Natriumhydrid, Kaliumhydroxid.

Bevorzugt wird der Sauerstoff als reiner Sauerstoff oder eine Sauerstoff enthaltende Mischung, wie zum Beispiel Luft oder mit Sauerstoff angereicherte Luft verwendet.

Bevorzugt wird der Sauerstoff in From von Sauerstoffbildnern wie zum Beispiel Wasserstoffperoxid verwendet.

Bevorzugt ist das Verhältnis von Sauerstoff zu phosphorhaltiger Verbindung (VI) oder (VI') 1:1 bis 1500:1.

Die mono-carboxyfunktionalisierten Dialkylphosphinsäure oder deren Salz (III) kann im Folgenden zu weiteren Metallsalzen umgesetzt werden.

Bevorzugt handelt es sich bei den eingesetzten Metallverbindungen der Verfahrensstufe d) um Verbindungen der Metalle Mg, Ca, Al, Sb, Sn, Ge, Ti, Fe, Zr, Zn, Ce, Bi, Sr, Mn, Li, Na, K, besonders bevorzugt Mg, Ca, Al, Ti, Zn, Sn, Ce, Fe.

Geeignete Lösungsmittel für Verfahrensstufe d) sind die, wie sie weiter vorne in Verfahrensstufe a) eingesetzt werden.

Bevorzugt erfolgt die Umsetzung der in Verfahrensstufe d) in wässrigem Medium.

Bevorzugt setzt man in Verfahrensstufe d) die nach Verfahrensstufe c) erhaltenen erhaltene mono-carboxyfunktionalisierten Dialkylphosphinsäuren, deren Ester und/oder Alkalisalze (III) mit Metallverbindungen von Mg, Ca, Al, Zn, Ti, Sn, Zr, Ce oder Fe zu den mono-carboxyfunktionalisierten Dialkylphosphinsäuresalzen (III) dieser Metalle um.

Die Umsetzung erfolgt dabei in einem Molverhältnis von monocarboxyfunktionalisierter Dialkylphosphinsäure/-ester/-salz (III) zu Metall von 8 zu 1 bis 1 zu 3 (für vierwertige Metallionen oder Metalle mit stabiler vierwertiger Oxidationsstufe), von 6 zu 1 bis 1 zu 3 (für dreiwertige Metallionen oder Metalle mit stabiler dreiwertiger Oxidationsstufe), von 4 zu 1 bis 1 zu 3 (für zweiwertige Metallionen oder Metalle mit stabiler zweiwertiger Oxidationsstufe) und von 3 zu 1 bis 1 zu 4 (für einwertige Metallionen oder Metalle mit stabiler einwertiger Oxidationsstufe).

Bevorzugt führt man in Verfahrenstufe c) erhaltenes monocarboxyfunktionalisiertes Dialkylphosphinsäureester/-salz (III) in die entsprechende Dialkylphosphinsäure über und setzt in Verfahrensstufe d) diese mit Metallverbindungen von Mg, Ca, Al, Zn, Ti, Sn, Zr, Ce oder Fe zu den mono-carboxyfunktionalisierten Dialkylphosphinsäuresalzen (III) dieser Metalle um.

Bevorzugt wandelt man in Verfahrenstufe c) erhaltene mono-carboxyfunktionalisierte Dialkylphosphinsäure/-ester (III) in ein Dialkylphosphinsäure-Alkalisalz um und setzt in Verfahrensstufe d) dieses mit Metallverbindungen von Mg, Ca, Al, Zn, Ti, Sn, Zr, Ce oder Fe zu den mono-carboxyfunktionalisierten Dialkylphosphinsäuresalzen (III) dieser Metalle um.

Bevorzugt handelt es sich bei den Metallverbindungen von Mg, Ca, Al, Zn, Ti, Sn, Zr, Ce oder Fe für Verfahrenstufe d) um Metalle, Metalloxide, -hydroxide, -oxid-hydroxide, -borate, -carbonate, -hydroxocarbonate, -hydroxocarbonathydrate, gemischte -hydroxocarbonate, - gemischte hydroxocarbonathydrate, -phosphate, -sulfate, -sulfat hydrate, -hydroxosulfathydrate, gemischte -hydroxosulfathydrate, -oxysulfate, -acetate, -nitrate, fluoride, -fluoridhydrate, -chloride, chloridhydrate, -oxychloride, -bromide, -iodide, -iodid hydrate, -carbonsäurederivate und/oder -alkoxide.

Bevorzugt handelt es sich bei den Metallverbindungen um Aluminiumchlorid, Aluminiumhydroxid, Aluminiumnitrat, Aluminiumsulfat, Titanylsulfat, Zinknitrat, Zinkoxid, Zinkhydroxid und/oder Zinksulfat.

Geeignet sind auch metallisches Aluminium, -fluorid, -hydroxychlorid, -bromid, -iodid, -sulfid, -selenid; -phosphid, -hypophosphit,-antimonid, -nitrid; -carbid, -hexafluorosilicat; -hydrid, -calciumhydrid, -borhydrid; -chlorat; Natrium-Aluminiumsulfat, Aluminium-Kaliumsulfat, Aluminiumammoniumsulfat, -nitrat, -metaphosphat, -phosphat, -silicat, -magnesiumsilicat, -carbonat, -hydrotalcit, -natriumcarbonat, -borat; -thiocyanat; -oxid, -oxidhydroxid, ihre entsprechenden Hydrate und/oder Polyaluminiumhydroxy-verbindungen, die vorzugsweise einen Aluminiumgehalt von 9 bis 40 Gew.-% besitzen.

Geeignet sind auch Aluminiumsalze von Mono-, Di-, Oligo-, Polycarbonsäuren wie z. B. Aluminiumdiacetat, -acetotartrat, -formiat, -lactat, -oxalat, -tartrat, -oleat, -palmitat, -sterarat, -trifluoromethansulfonat, -benzoat,-salicylat, -8-oxychinolat.

Geeignet sind ebenfalls elementares, metallisches Zink sowie Zinksalze wie z. B. Zinkhalogenide (Zinkfluorid, Zinkchloride, Zinkbromid, Zinkiodid).

Geeignet ist auchZinkborat, -carbonat,-hydroxidcarbonat, -silicat, -hexafluorosilicat, -stannat, -hydroxidstannat, -Magnesium-Aluminium-Hydroxidcarbonat; -nitrat, -nitrit, -phosphat, -pyrophosphat; -sulfat, -phosphid, -selenid, -tellurid und Zinksalze der Oxosäuren der siebten Hauptgruppe (Hypohalogenite, Halogenite, Halogenate, z. B. Zinkiodat, Perhalogenate, z. B. Zinkperchlorat); Zinksalze der Pseudohalogenide (Zinkthiocyanat, -cyanat, -cyanid); Zinkoxide, -peroxide, -hydroxide oder gemischte Zinkoxidhydroxide.

Bevorzugt sind Zinksalze der Oxosäuren der Übergangsmetalle (bspw. Zinkchromat(VI)hydroxyd, -chromit, -molybdat, -permanganat, -molybdat).

Geeignet sind auch Zinksalze von Mono-, Di-, Oligo-, Polycarbonsäuren, wie z. B. Zinkformiat, -acetat, -trifluoracetat, -propionat, -butyrat, -valerat, -caprylat, -oleat, -stearat, -oxalat, -tartrat, -citrat, -benzoat, -salicylat, -lactat, -acrylat, -maleat, -succinat, Salze von Aminosäuren (Glyzin), von sauren Hydroxyfunktionen (Zinkphenolat etc.), Zink-p-phenolsulfonat, -acetylacetonat, -stannat, -dimethyldithiocarbamat, -trifluormethansulfonat.

Bei den Titan-Verbindungen ist metallisches Titan ebenso wie Titan(III) und/oder (IV) -chlorid, -nitrat, -sulfat, -formiat, -acetat, -bromid, -fluorid, -oxychlorid, -oxysulfat, -oxid, -n-propoxid, -n-butoxid, -isopropoxid, -ethoxid, -2-ethylhexyloxid.

Geeignet ist auch metallisches Zinn sowie Zinnsalze (Zinn(II) und /oder (IV) -chlorid); Zinnoxide und Zinn-Alkoxid wie z. B. Zinn-(IV)-tert-butoxid.

Geeignet sind auch Cer(III)fluorid, -chlorid, -nitrat.

Bei den Zirkonium-Verbindungen ist metallisches Zirkonium sowie Zirkoniumsalze wie Zirkoniumchlorid, -sulfat, Zirconylacetat, Zirconylchlorid bevorzugt. Weiterhin bevorzugt sind Zirkonoxide sowie Zirkon-(IV)-tert-butoxid.

Bevorzugt erfolgt die Umsetzung in Verfahrensstufe d) bei einem Feststoffgehalt der mono-carboxyfunktionalisierten Dialkylphosphinsäuresalze von 0,1 bis 70 Gew.-%, bevorzugt 5 bis 40 Gew.-%.

Bevorzugt erfolgt die Umsetzung in Verfahrensstufe d) bei einer Temperatur von 20 bis 250 °C, bevorzugt bei einer Temperatur von 80 bis 120 °C.

Bevorzugt erfolgt die Umsetzung in Verfahrensstufe d) bei einem Druck zwischen 0,01 und 1000 bar, bevorzugt 0.1 bis 100 bar.

Bevorzugt erfolgt die Umsetzung in Verfahrensstufe d) während einer Reaktionszeit von 1*10⁻⁷ bis 1000 h.

Bevorzugt wird das nach der Verfahrensstufe d) durch Filtrieren und/oder Zentrifugieren aus dem Reaktionsgemisch abgetrennte mono-carboxyfunktionalisierte Dialkylphosphinsäuresalz (III) getrocknet.

Bevorzugt wird das nach Verfahrensstufe c) erhaltene Produktgemisch ohne weitere Reinigung mit den Metallverbindungen umgesetzt.

Bevorzugte Lösungsmittel sind die in Verfahrensschritt a) genannten Lösungsmittel.

Bevorzugt ist die Umsetzung in Verfahrensstufe d), c) und/oder b) im durch Stufe a) gegebenen Lösungsmittelsystem.

Bevorzugt ist die Umsetzung in Verfahrensstufe d) in einem modifizierten gegebenen Lösungsmittelsystem. Hierfür werden acide Komponenten, Lösevermittler, Schauminhibitoren etc. zugegeben.

In einer weiteren Ausführungsform des Verfahrens wird das nach Verfahrensstufe a), b) und/oder c) erhaltene Produktgemisch aufgearbeitet.

In einer weiteren Ausführungsform des Verfahrens wird das nach Verfahrensstufe c) erhaltene Produktgemisch aufgearbeitet und danach die nach Verfahrensstufe c) erhaltenen mono-carboxyfunktionalisierten Dialkylphosphinsäuren und/oder deren Salze oder Ester (III) in Verfahrensstufe d) mit den Metallverbindungen umgesetzt.

Bevorzugt wird das Produktgemisch nach Verfahrensstufe c) aufgearbeitet, indem die mono-carboxyfunktionalisierten Dialkylphosphinsäuren und/oder deren Salze oder Ester (III) durch Entfernen des Lösungsmittelsystems isoliert werden, z. B. durch Eindampfen.

Bevorzugt weist das mono-carboxyfunktionalisierte Dialkylphosphinsäuresalz III der Metalle Mg, Ca, Al, Zn, Ti, Sn, Zr, Ce oder Fe wahlweise eine Restfeuchte von 0,01 bis 10 Gew.-%, bevorzugt von 0,1 bis 1 Gew.-%, eine mittlere Teilchengröße von 0,1 bis 2000 µm, bevorzugt von 10 bis 500 µm, eine Schüttdichte von 80 bis 800 g/l, bevorzugt von 200 bis 700 g/l, eine Rieselfähigkeit nach Pfrengle von 0,5 bis 10, bevorzugt von 1 bis 5, auf.

Besonders bevorzugt enthalten die Formkörper, -Filme, -Fäden und -Fasern 5 bis 30 Gew.-% der mono-carboxyfunktionalisierte Dialkylphosphinsäure/-ester/-salze, hergestellt nach einem oder mehreren der Ansprüche 1 bis 13, 5 bis 90 Gew.-% Polymer oder Mischungen derselben, 5 bis 40 Gew.-% Additive und 5 bis 40 Gew.-% Füllstoff, wobei die Summe der Komponenten immer 100 Gew.-% beträgt.

Bevorzugt handelt es sich bei den Additiven um Antioxidantien, Antistatica, Treibmittel, weitere Flammschutzmittel, Hitzestabilisatoren,
Schlagzähmodifikatoren, Prozesshilfsmittel, Gleitmittel, Lichtschutzmittel, Antidrippingmittel, Compatibilizer, Verstärkungsstoffe, Füllstoffe, Keimbildungsmittel, Nukleierungsmittel, Additive zur Lasermarkierung, Hydrolysestabilisatoren, Kettenverlängerer, Farbpigmente, Weichmacher und/oder Plastifizierungsmittel.

Bevorzugt ist ein Flammschutzmittel, enthaltend 0,1 bis 90 Gew.-% der mono-carboxyfunktionalisierten Dialkylphosphinsäure, -ester und -salze (III) und 0,1 bis 50 Gew.-% weitere Additive, besonders bevorzugt Diole.

Bevorzugte Additive sind auch Aluminiumtrihydrat, Antimonoxid, bromierte aromatische oder cycloaliphatische Kohlenwasserstoffe, Phenole, Ether, Chlorparaffin, Hexachlorocyclopentadien-Addukte, Roter Phosphor, Melaminderivate, Melamincyanurate, Ammoniumpolyphosphate und Magnesiumhydroxid. Bevorzugte Additive sind auch weitere Flammschutzmittel, insbesondere Salze von Dialkylphosphinsäuren.

Insbesondere betrifft die Erfindung die Verwendung der erfindungsgemäßen mono-carboxyfunktionalisierten Dialkylphosphinsäure, -ester und -salze (III) als Flammschutzmittel bzw. als Zwischenstufe zur Herstellung von Flammschutzmitteln für thermoplastische Polymere wie Polyester, Polystyrol oder Polyamid und für duroplastische Polymere wie ungesättigte Polyesterharze, Epoxidharze, Polyurethane oder Acrylate.

Geeignete Polyester leiten sich von Dicarbonsäuren und deren Ester und Diolen und/oder von Hydroxycarbonsäuren oder den entsprechenden Lactonen ab. Besonders bevorzugt wird Terephthalsäure und Ethylenglykol, Propan-1,3-diol und Butan-1,3-diol eingesetzt.

Geeignete Polyester sind u.a. Polyethylenterephthalat, Polybutylenterephthalat (Celanex^{®} 2500, Celanex^{®} 2002, Fa Celanese; Ultradur^{®}, Fa. BASF), Poly-1,4-dimethylolcyclohexanterephthalat, Polyhydroxybenzoate, sowie Block-Polyetherester, die sich von Polyethern mit Hydroxylendgruppen ableiten; ferner mit Polycarbonaten oder MBS modifizierte Polyester.

Synthetische lineare Polyester mit permanentem Flammschutz setzen sich aus Dicarbonsäure-Komponenten, Diol-Komponenten der erfindungsgemäßen mono-carboxyfunktionalisierten Dialkylphosphinsäuren und -ester oder aus der nach dem erfindungsgemäßen Verfahren hergestellten mono-carboxyfunktionalisierten Dialkylphosphinsäuren und -ester als Phosphor-enthaltende Kettenglieder zusammen. Die Phosphor enthaltenden Kettenglieder machen 2-20 % Gew.-% der Dicarbonsäure-Komponente des Polyesters aus. Bevorzugt beträgt der resultierende Phosphorgehalt im Polyester 0,1-5%, besonders bevorzugt 0,5-3% Gew.-%.

Die folgenden Schritte können mit oder unter Zugabe der erfindungsgemäß hergestellten Verbindungen ausgeführt werden.

Bevorzugt wird zur Herstellung der Formmasse ausgehend von den freien Dicarbonsäure und Diolen zunächst direkt verestert und dann polykondensiert.

Bevorzugt wird ausgehend von Dicarbonsäureestern, insbesondere Dimethylestern, zunächst umgeestert und dann unter Verwendung der hierfür üblichen Katalysatoren polykondensiert.

Bevorzugt können bei der Polyesterherstellung neben den gängigen Katalysatoren auch übliche Additive (Vernetzungsmittel, Mattierungs- und Stabilisierungsmittel, Nukleierungsmittel, Farb- und Füllstoffe etc.) zugesetzt werden.

Bevorzugt findet die Veresterung und/oder Umesterung bei der Polyesterherstellung bei Temperaturen von 100 - 300 °C statt, besonders bevorzugt bei 150 - 250 °C.

Bevorzugt findet die Polykondensation bei der Polyesterherstellung bei Drücken zwischen 0,1 bis 1,5 mbar und Temperaturen von 150 - 450 °C statt, besonders bevorzugt bei 200 - 300 °C.

Die erfindungsgemäß hergestellten flammgeschützten Polyester-Formmassen werden bevorzugt in Polyester-Formkörpern eingesetzt.

Bevorzugte Polyester-Formkörper sind Fäden, Fasern, Folien und Formkörper, die als Dicarbonsäure-Komponente hauptsächlich Terephthalsäure und als Diolkomponente hauptsächlich Ethylenglykol enthalten.

Bevorzugt beträgt der resultierende Phosphorgehalt in aus flammgeschützten Polyester hergestellten Fäden und Fasern 0,1-18, bevorzugt 0,5-15 und bei Folien 0,2 - 15, bevorzugt 0,9 - 12 Gew.-%.

Geeignete Polystyrole sind Polystyrol, Poly-(p-methylstyrol) und/oder Poly-(alpha-methylstyrol).

Bevorzugt handelt es sich bei den geeigneten Polystyrolen um Copolymere von Styrol oder alpha-Methylstyrol mit Dienen oder Acrylderivaten, wie z. B. Styrol-Butadien, Styrol-Acrylnitril, Styrol-Alkylmethacrylat, Styrol-Butadien-Alkylacrylat und -methacrylat, Styrol-Maleinsäureanhydrid, Styrol-Acrylnitril-Methylacrylat; Mischungen von hoher Schlagzähigkeit aus Styrol-Copolymeren und einem anderen Polymer, wie z. B. einem Polyacrylat, einem Dien-Polymeren oder einem Ethylen-Propylen-Dien-Terpolymeren; sowie Block-Copolymere des Styrols, wie z. B. Styrol-Butadien-Styrol, Styrol-Isopren-Styrol, Styrol-Ethylen/Butylen-Styrol oder Styrol-Ethylen/Propylen-Styrol.

Bevorzugt handelt es sich bei den geeeigneten Polystyrolen auch um Pfropfcopolymere von Styrol oder alpha-Methylstyrol, wie z. B. Styrol auf Polybutadien, Styrol auf Polybutadien-Styrol- oder Polybutadien-Acrylnitril-Copolymere, Styrol und Acrylnitril (bzw. Methacrylnitril) auf Polybutadien; Styrol, Acrylnitril und Methyl-methacrylat auf Polybutadien; Styrol und Maleinsäureanhydrid auf Polybutadien; Styrol, Acrylnitril und Maleinsäureanhydrid oder Maleinsäureimid auf Polybutadien; Styrol und Maleinsäureimid auf Polybutadien, Styrol und Alkylacrylate bzw. Alkylmethacrylate auf Polybutadien, Styrol und Acrylnitril auf Ethylen-Propylen-Dien-Terpolymeren, Styrol und Acrylnitril auf Polyalkylacrylaten oder Polyalkylmeth-acrylaten, Styrol und Acrylnitril auf Acrylat-Butadien-Copolymeren, sowie deren Mischungen, wie sie z. B. als so genannte ABS-, MBS-, ASA- oder AES-Polymere bekannt sind.

Bevorzugt handelt es sich bei den Polymeren um Polyamide und Copolyamide, die sich von Diaminen und Dicarbonsäuren und/oder von Aminocarbonsäuren oder den entsprechenden Lactamen ableiten, wie Polyamid 2,12, Polyamid 4, Polyamid 4,6, Polyamid 6, Polyamid 6,6, Polyamid 6,9, Polyamid 6,10, Polyamid 6,12, Polyamid 6,66, Polyamid 7,7, Polyamid 8,8, Polyamid 9,9, Polyamid 10,9, Polyamid 10,10 , Polyamid 11, Polyamid 12, usw. Solche Polyamide sind z. B unter den Handelsnamen Nylon^{®}, Fa. DuPont, Ultramid^{®}, Fa. BASF, Akulon^{®} K122, Fa. DSM, Zytel^{®} 7301, Fa. DuPont; Durethan^{®} B 29, Fa. Bayer und Grillamid^{®}, Fa. Ems Chemie bekannt.

Geeignet sind auch aromatische Polyamide ausgehend von m-Xylol, Diamin und Adipinsäure; Polyamide, hergestellt aus Hexamethylendiamin und Iso- und/oder Terephthalsäure und gegebenenfalls einem Elastomer als Modifikator, z. B. Poly-2,4,4-trimethylhexamethylen-terephthalamid oder Poly-m-phenylenisophthalamid, Blockcopolymere der vorstehend genannten Polyamide mit Polyolefinen, Olefin-Copolymeren, lonomeren oder chemisch gebundenen oder gepfropften Elastomeren, oder mit Polyethern, wie z. B. mit Polyethylenglykol, Polypropylenglykol oder Polytetramethylenglykol. Ferner mit EPDM oder ABS modifizierte Polyamide oder Copolyamide; sowie während der Verarbeitung kondensierte Polyamide ("RIM-Polyamidsysteme").

Die mono-carboxyfunktionalisierte Dialkylphosphinsäure/-ester/-salze, hergestellt nach einem oder mehreren der Ansprüche 1 bis 13 werden bevorzugt in Formmassen angewendet, die weiter zur Erzeugung von Polymer-Formkörpern eingesetzt werden.

Besonders bevorzugt enthält die flammgeschützte Formmasse 5 bis 30 Gew.-% mono-carboxyfunktionalisierte Dialkylphosphinsäuren, -salze oder -ester, die nach einem oder mehreren der Ansprüche 1 bis 13 hergestellt wurden, 5 bis 90 Gew.-% Polymer oder Mischungen derselben, 5 bis 40 Gew.-% Additive und 5 bis 40 Gew.-% Füllstoff, wobei die Summe der Komponenten immer 100 Gew.-% beträgt.

Die Erfindung betrifft auch Flammschutzmittel, die die mono-carboxyfunktionalisierte Dialkylphosphinsäuren, -salze oder -ester, die nach einem oder mehreren der Ansprüche 1 bis 13 hergestellt wurden enthalten.

Außerdem betrifft die Erfindung Polymer-Formmassen sowie Polymer-Formkörper, -Filme, -Fäden und -Fasern, enthaltend die erfindungsgemäß hergestellten mono-carboxyfunktionalisierten Dialkylphosphinsäuresalze (III) der Metalle Mg, Ca, Al, Zn, Ti, Sn, Zr, Ce oder Fe.

Die Erfindung wird durch die nachstenden Beispiele erläutert.

Herstellung, Verarbeitung und Prüfung von flammgeschützten Polymerformmassen und flammgeschützten Polymerformkörpern

Die Flammschutzkomponenten werden mit dem Polymergranulat und evtl. Additiven vermischt und auf einem Doppelschnecken-Extruder (Typ Leistritz LSM^{®} 30/34) bei Temperaturen von 230 bis 260 °C (PBT-GV) bzw. von 260 bis 280 °C (PA 66-GV) eingearbeitet. Der homogenisierte Polymerstrang wurde abgezogen, im Wasserbad gekühlt und anschließend granuliert.

Nach ausreichender Trocknung wurden die Formmassen auf einer Spritzgießmaschine (Typ Aarburg Allrounder) bei Massetemperaturen von 240 bis 270 °C (PBT-GV) bzw. von 260 bis 290 °C (PA 66-GV) zu Prüfkörpern verarbeitet. Die Prüfkörper werden anhand des UL 94-Tests (Underwriter Laboratories) auf Flammwidrigkeit (Flammschutz) geprüft und klassifiziert.

An Prüfkörpern aus jeder Mischung wurden die Brandklasse UL 94 (Underwriter Laboratories) an Probekörpern der Dicke 1,5 mm bestimmt.

Nach UL 94 ergeben sich folgende Brandklassen:
V-0: kein Nachbrennen länger als 10 sec, Summe der Nachbrennzeiten bei 10 Beflammungen nicht größer als 50 sec, kein brennendes Abtropfen, kein vollständiges Abbrennen der Probe, kein Nachglühen der Proben länger als 30 sec nach Beflammungsende
V-1: kein Nachbrennen länger als 30 sec nach Beflammungsende, Summe der Nachbrennzeiten bei 10 Beflammungen nicht größer als 250 sec, kein Nachglühen der Proben länger als 60 sec nach Beflammungsende, übrige Kriterien wie bei V-0 V-2: Zündung der Watte durch brennendes Abtropfen, übrige Kriterien wie bei V-1 Nicht klassifizierbar (nkl): erfüllt nicht die Brandklasse V-2.

Bei einigen untersuchten Proben wurde außerdem der LOI-Wert gemessen. Der LOI-Wert (Limiting Oxygen Index) wird nach ISO 4589 bestimmt. Nach ISO 4589 entspricht der LOI der geringsten Sauerstoffkonzentration in Volumenprozent, die in einer Mischung von Sauerstoff und Stickstoff gerade noch die Verbrennung des Kunststoffs unterhält. Je höher der LOI-Wert, desto schwerer entflammbar ist das geprüfte Material.

| | | |
|---|---|---|
| LOI | 23 | brennbar |
| LOI | 24-28 | bedingt brennbar |
| LOI | 29-35 | flammwidrig |
| LOI | >36 | besonders flammwidrig |

### Eingesetzte Chemikalien und Abkürzungen

| | |
|---|---|
| VE-Wasser | voll-entsalztes Wasser |
| AIBN | Azo-bis-(isobutyronitril), (Fa. WAKO Chemicals GmbH) |
| WakoV65 | 2,2'-Azobis(2,4-dimethyl-valeronitril), (Fa. WAKO Chemicals GmbH) |
| Deloxan^{®} THP II | Metallfänger (Fa. Evonik Industries AG) |

### Beispiel 1

Bei Raumtemperatur werden in einem Dreihalskolben mit Rührer und Intensivkühler 188 g Wasser vorgelegt und unter Rühren und Durchleiten von Stickstoff entgast. Dann werden unter Stickstoff 0,2 mg Palladium(II)sulfat und 2,3 mg Tris(3-sulfophenyl)-phosphin Trinatriumsalz hinzugegeben und gerührt, dann 66 g Phosphinsäure in 66 g Wasser zugegeben. Die Reaktionslösung wird in einen 2 I-Büchi-Reaktor überführt und unter Rühren und unter Druck mit Ethylen beschickt und das Reaktionsgemisch auf 80 °C geheizt. Nach einer Ethylenaufnahme von 28 g wird abgekühlt und freies Ethylen abgelassen. Das Reaktionsgemisch wird am Rotationsverdampfer vom Lösungsmittel befreit. Der Rückstand wird mit 100 g VE-Wasser versetzt und bei Raumtemperatur unter Stickstoffatmosphäre gerührt, dann filtriert und das Filtrat mit Toluol extrahiert, danach wird am Rotationsverdampfer vom Lösungsmittel befreit und die erhaltene Ethylphosphonigsäure [92 g (98 % der Theorie)] aufgefangen.

### Beispiel 2

Wie in Beispiel 1 werden 99 g Phosphinsäure, 396 g Butanol, 42 g Ethylen, 6,9 mg Tris(dibenzylidenaceton)dipalladium, 9,5 mg 4,5-Bis(diphenylphosphino)-9,9-dimethylxanthen umgesetzt, dann zur Reinigung über eine mit Deloxan^{®} THP II beschickte Säule gegeben und danach nochmal n-Butanol zugegeben. Bei einer Reaktionstemperatur von 80 - 110 °C wird das gebildete Wasser durch Azeotropdestillation entfernt. Das Produkt wird durch Destillation bei vermindertem Druck gereinigt. Ausbeute: 189 g (84 % der Theorie) Ethylphosphonigsäurebutylester.

### Beispiel 3

Wie in Beispiel 1 werden 198 g Phosphinsäure, 198 g Wasser, 84 g Ethylen, 6,1 mg Palladium(II)sulfat, 25,8 mg 9,9-Dimethyl-4,5-bis(diphenylphosphino)-2,7-sulfonato-xanthen Dinatriumsalz umgesetzt, dann zur Reinigung über eine mit Deloxan^{®} THP 11 beschickte Säule gegeben und danach n-Butanol zugegeben. Bei einer Reaktions-temperatur von 80 - 110 °C wird das gebildete Wasser durch Azeotropdestillation entfernt. Das Produkt wird durch Destillation bei vermindertem Druck gereinigt. Man erhält so 374 g (83 % der Theorie) Ethylphosphonigsäurebutylester.

### Beispiel 4

In einem 500 ml-Fünfhalskolben mit Gaseinleitungsrohr, Thermometer, Intensivrührer und Rückflusskühler mit Gasverbrennung werden 94 g (1 mol) Ethylphosphonigsäure (hergestellt wie in Beispiel 1) vorgelegt. Bei Raumtemperatur wird Ethylenoxid eingeleitet. Unter Kühlung wird eine Reaktionstemperatur von 70 °C eingestellt und noch eine Stunde bei 80 °C nachreagiert. Die Ethylenoxidaufnahme beträgt 65,7 g. Die Säurezahl des Produktes ist kleiner 1 mg KOH/g. Es werden 129 g (94 % der Theorie) (Ethylphosphonigsäure-2-hydroxyethylester) als farbloses, wasserklares Produkt erhalten.

### Beispiel 5

564 g (6 mol) Ethylphosphonigsäure (hergestellt wie in Beispiel 1) werden in 860 g Wasser gelöst und in einen 51 Fünfhalskolben mit Thermometer, Rückflusskühler, Intensivrührer und Tropftrichter vorgelegt. Nach Aufheizen der Reaktionsmischung auf 100 °C wird bei Normaldruck innerhalb von 1 h 406 g (7 mol) Allylalkohol und 500 g einer 5 %igen Natriumperoxodisulfat-Lösung (1,5 mol-% bzgl. Allylalkohol) zugetropft. Anschliessend wird das Wasser im Vakuum abdestilliert. Der Rückstand wird in Tetrahydrofuran aufgenommen und die unlöslichen Salze abfiltriert. Das Lösungsmittel des Filtrats wird im Vakuum abgetrennt. Es werden 812 g (89 % der Theorie) Ethyl-3-hydroxypropylphosphinsäure als Öl erhalten.

### Beispiel 6

94 g (1 mol) Ethylphosphonigsäure (hergestellt wie in Beispiel 1) und 114 g (1 mol) 2-Methyl-2-propen-1-ol werden in einen Vierhalsrundkolben mit Rührer, Rückflusskühler, Thermometer und Stickstoffeinlass in 200 ml Eisessig vorgelegt und erwärmt. Innerhalb von 1 h werden bei ca. 100 °C 98,4 g einer 5 %-igen Lösung von AIBN in Eisessig zugetropft. Danach wurde das Lösungmittel im Vakuum abdestilliert. Es werden 153 g Ethyl-(2-methyl-3-hydroxypropyl)-phosphinsäure erhalten.

### Beispiel 7

In einem 11 Fünfhalskolben mit Thermometer, Rückflusskühler, Intensivrührer und Tropftrichter wurden 447 g (3 mol) Ethylphosphonigsäurebutylester (hergestellt wie in Beispiel 3) und 168 g (3 mol) 2-Propenal vorgelegt. Unter Rühren werden 15 ml Natriumbutylat (30 %ig in Butanol) in einer solchen Geschwindigkeit dazugetropft, dass sich eine Reaktionstemperatur von max. 120 °C einstellt. Das so erhaltene Rohprodukt wird im Vakuum destilliert. Es wurden 550 g (89 % der Theorie) Ethyl-(2-formylethyl)-phosphinsäurebutylester als farblose Flüssigkeit erhalten.

### Beispiel 8

In einem 500 ml-Fünfhalskolben mit Thermometer, Rückflusskühler, Intensivrührer und Tropftrichter werden 63,5 g (0,46 mol) Ethylphosphonigsäure-2-hydroxyethylester (hergestellt wie in Beispiel 4) und 32,2 g (0,46 mol) 2-Methyl-2-propenal vorgelegt. Unter Rühren werden 25 ml Natriumethylat (30 %ig in Ethanol) in einer solcher Geschwindigkeit dazugetropft, dass sich eine Reaktionstemperatur von 60 °C einstellt. Es wird eine schwach gelb gefärbte Flüssigkeit erhalten. Das so erhaltene Rohprodukt wird im Vakuum destilliert. Ausbeute: 84 g (88 % der Theorie) Ethyl-(2-methyl-2-formylethyl)-phosphinsäure-2-hydroxyethylester als farblose Flüssigkeit.

### Beispiel 9

Ein Umlaufreaktor mit einem Inhalt von 1 L wird mit einem Gemisch aus 801 g (4,5 mol) Ethyl-(2-formylethyl)-phosphinsäureethylester (hergestellt analog Beispiel 7) und 62 g (1,35 Mol) Ethanol gefüllt. Nach Inbetriebnahme der Pumpe werden stündlich ein Gemisch von 726 g (6,00 Mol)
Ethyphosphonigsäureethylester und 336 g (6,00 Mol) 2-Propenal sowie eine Lösung von 16,8 g (0,20 Mol) Kaliumethylat in 120 g (2,61 Mol) Ethanol eindosiert, wobei mit Hilfe des Kühlwasserkreislaufes eine Temperatur der Reaktionsmischung von ca. 40 °C eingehalten wurde. Das überlaufende Rohprodukt wird 30 Stunden gesammelt und ergab mit dem aus dem Reaktor abgelassenen Produkt eine Gesamtmenge von 35,5 kg. Nach Abtrennung der Leichtsieder durch Destillation unter Wasserstrahlvakuum und Filtration wurde das Produkt in einem Dünnschichtverdampfer unter Vakuum destilliert und es wurden 30,0 kg (168,6 Mol) Ethyl-(2-formylethyl)-phosphinsäureethylester erhalten. Dies entspricht nach Abzug der im Reaktor vorgelegten Menge einer P-Ausbeute von 93,8 % bei einer Leistung von ca. 1000 g/l*h.Somit ist eine kontinuierliche Produktion von mono-2-formyl-funktionalisierten Dialkylphosphinsäureestern in guten Raum-Zeit-Ausbeuten möglich.

### Beispiel 10

149 g (1 mol) Ethylphosphonigsäurebutylester (hergestellt wie in Beispiel 2) und 67 g (1,2 mol) 2-Propenal in 217 g Toluol werden auf ca. 100 °C erwärmt. Unter Rühren werden 124 g einer 10 %-igen Lösung von WakoV65 in Toluol zudosiert. Das Lösungsmittel wird im Vakuum abdestilliert. Es werden 171 g (78 % der Theorie) Ethyl-(2-formylethyl)-phosphinsäurebutylester erhalten.

### Beispiel 11

15,2 g (0,1 mol) Ethyl-3-hydroxypropylphosphinsäure (hergestellt wie in Beispiel 5) werden in 150 ml Wasser gelöst und mit 2N NaOH-Lösung auf pH 9 gebracht. Anschließend werden 0,45 g Aktivkohle mit 5 % Pt und 1 % Bi hinzugefügt, die Suspension auf 70 °C erwärmt und Luft (10 l/h) durch die Suspension geleitet. Hierbei wird pH der Suspension durch Zugabe von 2N NaOH-Lösung auf pH = 9 gehalten. Nach Beendigung der Reaktion wird die Reaktionslösung von dem Katalysator filtriert, gewaschen und das Wasser im Vakuum abdestilliert. Es werden 19,5 g (93 % der Theorie) 3-(Ethylhydroxyphosphinyl)-propionsäure Natriumsalz als farbloser Feststoff erhalten.

### Beispiel 12

15,2 g (0,1 mol) Ethyl-3-hydroxypropylphosphinsäure (hergestellt wie in Beispiel 5) werden in 150 ml Wasser gelöst und mit 2N NaOH-Lösung auf pH 9 gebracht. Anschliessend werden 0,25 g Aktivkohle mit 5 % Pd und 1 % Bi hinzugefügt, die Suspension auf 70 °C erwärmt und 30 %ige Wasserstoffperoxidlösung mit einer Flussrate von 1 Moläquivalent pro Stunde in die Suspension geleitet und dabei der pH der Suspension durch Zugabe von 2N NaOH-Lösung auf pH = 9 gehalten. Nach Beendigung der Reaktion wird die Reaktionslösung von dem Katalysator filtriert, gewaschen und das Wasser im Vakuum abdestilliert. Ausbeute: 19,3 g (92 % der Theorie) 3-(Ethylhydroxyphosphinyl)-propionsäure Natriumsalz (farbloser Feststoff).

### Beispiel 13

20,6 g (0,1 mol) Ethyl-(2-formylethyl)-phosphinsäurebutylester (hergestellt wie in Beispiel 10) in 500 ml Aceton wurden mit 0,11 mol Jones-Reagenz (12,7 g Chromtrioxid in 36,7 ml Wasser und 11,0 ml konz. Schwefelsäure) bei 0 °C tropfenweise versetzt. Die Reaktionsmischung wurde noch 3 1/2 Stunden bei Eiskühlung und 1 Stunde bei Raumtemperatur gerührt. Nach Zugabe von 12 ml Isopropanol wird auf Eis/Wasser gegeben. Anschließend werden leichtflüchtige Bestandteile im Vakuum abdestilliert. Der Rückstand wurde in Tetrahydrofuran aufgenommen und extrahiert. Unlöslichen Salze wurden abfiltriert. Das Lösungsmittel des Filtrats wurde im Vakuum abgetrennt und der Rückstand aus Aceton umkristallisiert. Es wurden 17,3 g (78 % der Theorie) 3-(Ethylbutoxyphosphinyl)-propionsäure als Öl erhalten

### Beispiel 14

Eine wässrige Lösung von 420 g (2 mol) 3-(Ethylhydroxyphosphinyl)-propionsäure Natriumsalz (hergestellt wie in Beispiel 12) wird mit ca. 196 g konzentrierter Schwefelsäure sauer gestellt und das Wasser im Vakuum abdestilliert. Der Rückstand wurde in Tetrahydrofuran aufgenommen und extrahiert. Die unlöslichen Salze wurden abfiltriert. Das Lösungsmittel des Filtrats wurde im Vakuum abgetrennt und der Rückstand aus Aceton umkristallisiert. Es wurden 325 g (98 % der Theorie) 3-(Ethylhydroxyphosphinyl)-propionsäure als farbloser Feststoff erhalten.

### Beispiel 15

444 g (2 mol) 3-(Ethylbutoxyphosphinyl)-propionsäure (hergestellt wie in Beispiel 13) werden in einem 1 l Fünfhalskolben mit Thermometer, Rückflusskühler, Intensivrührer und Tropftrichter vorgelegt. Bei 160 °C wird während 4 h 500 ml Wasser eindosiert und eine Butanol-Wasser Mischung abdestilliert. Der feste Rückstand wird aus Aceton umkristallisiert. Es werden 309 g (93 % der Theorie) 3-(Ethylhydroxyphosphinyl)-propionsäure als farbloser Feststoff erhalten.

### Beispiel 16

996 g (6 mol) 3-(Ethylhydroxyphosphinyl)-propionsäure (hergestellt wie in Beispiel 14) werden in 860 g Wasser gelöst und in einem 51 Fünfhalskolben mit Thermometer, Rückflusskühler, Intensivrührer und Tropftrichter vorgelegt und mit ca. 960 g (12 mol) 50 %ige Natriumhydroxid-Lösung neutralisiert. Bei 85 °C wird eine Mischung von 2583 g einer 46 %igen wässrigen Lösung von Al₂(SO₄)₃·14 H₂O zugefügt. Anschließend wird der erhaltene Feststoff abfiltriert, mit heißem Wasser gewaschen und bei 130 °C im Vakuum getrocknet. Ausbeute: 1026 g (94 % der Theorie) 3-(Ethylhydroxy-phosphinyl)-propionsäure Aluminium(III)salz als farbloses Salz.

### Beispiel 17

222 g (1 mol) 3-(Ethylbutoxyphosphinyl)-propionsäure (hergestellt wie in Beispiel 13) und 85 g Titantetrabutylat werden in 500 ml Toluol 40 Stunden unter Rückfluss erhitzt. Dabei entstehendes Butanol wird mit Anteilen an Toluol von Zeit zu Zeit abdestilliert. Die entstandene Lösung wird anschließend vom Lösungsmittel befreit. Man erhält 227 g 3-(Ethylbutoxyphosphinyl)-propionsäure Titansalz.

### Beispiel 18

498 g (3 mol) 3-(Ethylhydroxyphosphinyl)-propionsäure (hergestellt wie in Beispiel 14) werden bei 85 °C in 400 ml Toluol gelöst und mit 888 g (12 mol) Butanol versetzt. Bei einer Reaktionstemperatur von ca. 100 °C wird das gebildete Wasser durch Azeotropdestillation entfernt. Das Produkt 3-(Ethylbutoxyphosphinyl)-propionsäurebutylester wird durch Destillation bei vermindertem Druck gereinigt.

### Beispiel 19

540 g (3,0 mol) 3-(Ethylhydroxyphosphinyl)-2-methylpropionsäure (hergestellt analog Beispiel 13) werden bei 80 °C in 400 ml Toluol gelöst und mit 594 g (6,6 mol) 1,4-Butandiol versetzt und in einer Destillationsapparatur mit Wasserabscheider bei ca. 100 °C während 4h verestert. Nach beendeter Veresterung wird das Toluol im Vakuum abgetrennt. Es werden 894 g (92 % der Theorie) 3-(Ethyl-4-hydroxy-butylphosphinyl)-2-methylpropionsäure-4-hydroxybutylester als farbloses Öl erhalten.

### Beispiel 20

Zu 276 g (1 mol) 3-(Ethylbutoxyphosphinyl)-propionsäurebutylester (hergestellt wie in Beispiel 18) werden 155 g (2,5 mol) Ethylenglycol und 0,4 g Kaliumtitanyloxalat hinzugegeben und 2 h bei 200 °C gerührt. Durch langsames Evakuieren werden leicht flüchtige Anteile abdestilliert. Es werden 244 g (98 % der Theorie) 3-(Ethyl-2-hydroxyethoxyphosphinyl)-propionsäure-2-hydroxyethylester erhalten.

### Beispiel 21

Es werden zu 25,4 g 3-(Ethyl-2-hydroxyethoxyphosphinyl)-propionsäure-2-hydroxyethylester 290 g Terephthalsäure, 188 g Ethylenglycol, 0,34 g Zinkacetat gegeben und 2 h auf 200 °C erhitzt. Dann werden 0,29 g Trinatriumphosphatanhydrat und 0,14 g Antimon(III)oxid hinzugegeben, auf 280 °C erhitzt und danach evakuiert. Aus der erhaltenen Schmelze (357 g, Phosphorgehalt 0.9 %) werden Probekörper der Dicke 1,6 mm für die Messung des Sauerstoffindexes (LOI) nach ISO 4589- 2 als auch für den Brandtest UL 94 (Underwriter Laboratories) gespritzt. Die so hergestellten Probekörper ergaben einen LOI von 42 % O₂ und erfüllten nach UL 94 die Brandklasse V-0. Entsprechende Probekörper ohne 3-(Ethyl-2-hydroxyethoxyphosphinyl)-propionsäure-2-hydroxyethylester ergaben einen LOI von nur 31 % O₂ und erfüllten nach UL 94 nur die Brandklasse V-2. Der 3-(Ethyl-2-hydroxyethoxyphosphinyl)-propionsäure-2-hydroxyethylester enthaltende Polyester-Formkörper zeigt damit eindeutig flammschützende Eigenschaften.

### Beispiel 22

Zu 15,2 g 3-(Ethylhydroxyphosphinyl)-2-methylpropionsäure (hergestellt analog Beispiel 13) werden 12,9 g 1,3-Propylenglycol zugegeben und bei 160 °C das bei der Veresterung gebildete Wasser abgezogen. Dann werden 378 g Dimethylterephthalat, 152 g 1,3-Propandiol, 0,22 g Tetrabutylitanat und 0,05 g Lithiumacetat zugegeben, die Mischung zunächst 2 h unter Rühren auf 130 bis 180 °C erhitzt, danach bei Unterdruck auf 270 °C. Das Polymer (438 g) enthält 0,6 % Phosphor, der LOI beträgt 34.

### Beispiel 23

Zu 14 g 3-(Ethylhydroxyphosphinyl)-propionsäure (hergestellt wie in Beispiel 14) werden 367 g Dimethylterephthalat, 170 g 1,4-Butandiol, 0,22 g Tetrabutylitanat und 0,05 g Lithiumacetat zugegeben, die Mischung zunächst 2 h lang unter Rühren auf 130 bis 180 °C erhitzt, danach bei Unterdruck auf 270 °C. Das Polymer (427 g) enthält 0,6 % Phosphor, der LOI beträgt 34 (unbehandeltes Polybutylenterephthalat: 23).

### Beispiel 24

In einem 250 ml Fünfhalskolben mit Rückflusskühler, Rührer, Thermometer und Stickstoffeinleitung werden 100 g eines Bisphenol-A-bisglycidethers mit einem Epoxidwert von 0,55 mol/100 g (Beckopox EP 140, Fa. Solutia) und 24.1 g (0,13 mol) 3-(Ethylhydroxyphosphinyl)-2-methylpropionsäure (hergestellt analog Beispiel 13) unter Rühren auf maximal 150 °C erhitzt. Nach 30 min ergibt sich eine klare Schmelze. Nach einer weiteren Stunde Rühren bei 150 °C wird die Schmelze abgekühlt und gemörsert. Man erhält 118,5 g eines weißen Pulvers mit einem Phosphorgehalt von 3,3 Gew.-%.

### Beispiel 25

In einem 2L-Kolben mit Rührer, Wasserabscheider, Thermometer, Rückflusskühler und Stickstoffeinleitung werden 29,4 g Phthalsäureanhydrid, 19,6 g Maleinsäure-anhydrid, 24,8 g Propylenglycol, 18,7 g 3-(Ethyl-2-hydroxyethylphosphinyl)-propion-säure-2-hydroxyethylester (hergestellt wie in Beispiel 20) 20 g Xylol und 50 mg Hydrochinon unter Rühren und Durchleiten von Stickstoff auf 100 °C erhitzt. Bei Einsetzen der exothermen Reaktionwird die Heizung entfernt. Nach Abklingen der Reaktion wird weiter bei ca. 190 °C gerührt. Nachdem 14 g Wasser abgeschieden sind, wird das Xylol abdestilliert und die Polymerschmelze abgekühlt. Man erhält 91,5 g eines weißen Pulvers mit einem Phosphorgehalt von 2,3 Gew.-%.

### Beispiel 26

Eine Mischung von 50 Gew.-% Polybutylenterephthalat, 20 Gew.-% 3-(Ethylhydroxy-phosphinyl)-propionsäure Aluminium(III)salz (hergestellt wie in Beispiel 16) und 30 Gew.-% Glasfasern werden auf einem DoppelschneckenExtruder (Typ Leistritz LSM 30/34) bei Temperaturen von 230 bis 260 °C zu einer Polymerformmasse compoundiert. Der homogenisierte Polymerstrang wurde abgezogen, im Wasserbad gekühlt und anschließend granuliert. Nach Trocknung werden die Formmassen auf einer Spritzgießmaschine (Typ Aarburg Allrounder) bei 240 bis 270 °C zu Polymerformkörper verarbeitet und eine UL-94 Klassifizierung von V-0 bestimmt.

### Beispiel 27

Eine Mischung von 53 Gew.-% Polyamid 6.6, 30 Gew.% Glasfasern, 17 Gew.-% 3-(Ethylbutoxyphosphinyl)-propionsäure Titansalz (hergestellt wie in Beispiel 17) werden auf einem Doppelschnecken-Extruder (Typ Leistritz LSM 30/34) zu Polymerformmassen compoundiert. Der homogenisierte Polymerstrang wurde abgezogen, im Wasserbad gekühlt und anschließend granuliert. Nach Trocknung werden die Formmassen auf einer Spritzgießmaschine (Typ Aarburg Allrounder) bei 260 bis 290°C zu Polymerformkörpern verarbeitet und eine UL-94 Klassifizierung von V-0 erhalten.

## Patentansprüche

1. Verfahren zur Herstellung von mono-carboxyfunktionalisierten Dialkylphosphin-säuren, -estern und -salzen, **dadurch gekennzeichnet, dass** man
a) eine Phosphinsäurequelle (I) 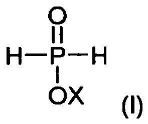 mit Olefinen (IV) 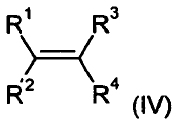 in Gegenwart eines Katalysators A zu einer Alkylphosphonigsäure, deren Salz oder Ester (II) 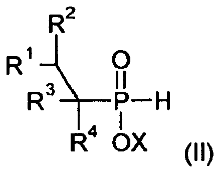 umsetzt,
b) die so entstandene Alkylphosphonigsäure, deren Salz oder Ester (II) mit einem
Allylalkohol (V) und/oder Acrolein (V') 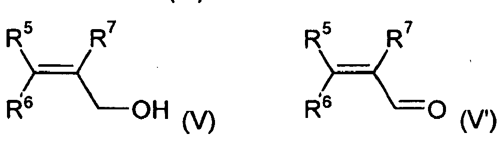 in Gegenwart eines Katalysators B zum mono-funktionalisierten Dialkylphosphinsäurederivat (VI) und/oder (VI') umsetzt und 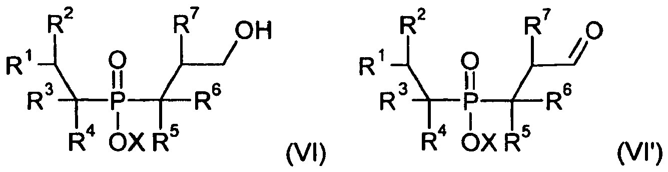
c) das mono-funktionalisierten Dialkylphosphinsäurederivat (VI) und/oder (VI') mit einem Oxidationsmittel oder mit einem Oxidationsmittel und Wasser oder in Gegenwart eines Katalysators C mit Sauerstoff und Wasser zum mono-carboxyfunktionalisierten Dialkylphosphinsäurederivat (III) 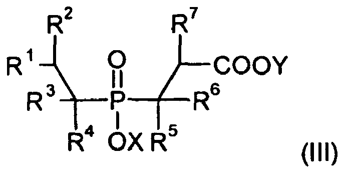 umsetzt oder die nach Schritt a) erhaltene Alkylphosphonigsäure, deren Salz oder Ester (II) und/oder die nach Schritt b) erhaltene mono-funktionalisierte Dialkylphosphinsäure, deren Salz oder Ester (VI) und/oder (VI') und/oder die nach Schritt c) erhaltene mono-carboxyfunktionalisierte Dialkylphosphinsäure, deren Salz oder Ester (III) und/oder die jeweils resultierende Reaktionslösung davon mit einem Alkylenoxid oder einem Alkohol M-OH und/oder M'-OH verestert, und den jeweils entstandenen Alkylphosphonigsäureester (II), monofunktionalisierten Dialkylphosphinsäurester (VI) und/oder (VI') und/oder monocarboxyfunktionalisierten Dialkylphosphinsäureester (III) den weiteren Reaktionsschritten b) oder c) unterwirft, wobei R¹, R², R³, R⁴, R⁵, R⁶, R⁷ gleich oder verschieden sind und unabhängig voneinander H, C₁-C₁₈-Alkyl, C₆-C₁₈-Aryl, C₆-C₁₈-Aralkyl, C₆-C₁₈-Alkyl-Aryl, CN, CHO, OC(O)CH₂CN, CH(OH)C₂H₅, CH₂CH(OH)CH₃, 9-Anthracen, 2-Pyrrolidon, (CH₂)ₘOH, (CH₂)ₘNH₂, (CH₂)ₘNCS, (CH₂)ₘNC(S)NH₂, (CH₂)ₘSH, (CH₂)ₘS-2-thiazolin, (CH₂)ₘSiMe₃, C(O)R⁸, CH=CH-R⁸, CH=CH-C(O)R⁸ bedeuten, wobei die Gruppen C₆-C₁₈-Aryl, C₆-C₁₈-Aralkyl und C₆-C₁₈-Alkyl-Aryl mit -C(O)CH₃, OH, CH₂OH, NH₂, NO₂, OCH₃, SH und/oder OC(O)CH₃ substituiert sein können und wobei R⁸ für C₁-C₈-Alkyl oder C₆-C₁₈-Aryl steht und m eine ganze Zahl von 0 bis 10 bedeutet und X und Y gleich oder verschieden sind und unabhängig voneinander für H, C₁-C₁₈-Alkyl, C₆-C₁₈-Aryl, C₆-C₁₈-Aralkyl, C₆-C₁₈-Alkyl-Aryl, (CH₂)ₖOH, CH₂-CHOH-CH₂OH, (CH₂)ₖO(CH₂)ₖH, (CH₂)ₖ-CH(OH)-(CH₂)ₖH, (CH₂-CH₂O)ₖH, (CH₂-C[CH₃]HO)ₖH, (CH₂-C[CH₃]HO)ₖ(CH₂-CH₂O)ₖH, (CH₂-CH₂O)ₖ(CH₂-C[CH₃]HO)H, (CH₂-CH₂O)ₖ-alkyl, (CH₂-C[CH₃]HO)ₖ-alkyl, (CH₂-C[CH₃]HO)ₖ(CH₂-CH₂O)ₖ-alkyl, (CH₂-CH₂O)ₖ(CH₂-C[CH₃]HO)O-alkyl, (CH₂)ₖ-CH=CH(CH₂)ₖH, (CH₂)ₖNH₂, (CH₂)ₖN[(CH₂)ₖH]₂ stehen wobei k eine ganze Zahl von 0 bis 10 ist und/oder für Mg, Ca, Al, Sb, Sn, Ge, Ti, Fe, Zr, Zn, Ce, Bi, Sr, Mn, Cu, Ni, Li, Na, K, H und/oder eine protonierte Stickstoffbase steht und es sich bei den Katalysatoren A und C um Übergangsmetalle und/oder Übergangsmetallverbindungen und/oder Katalysatorsysteme handelt, die sich aus einem Übergangsmetall und/oder einer Übergangsmetallverbindung und mindestens einem Liganden zusammensetzen und es sich bei dem Katalysator B um Peroxide bildende Verbindungen und/oder Peroxoverbindungen und/oder um Azo-Verbindungen und/oder um Alkali- und/oder Erdalkalimetalle, -hydride und/oder -alkoholate handelt.

2. Verfahren nach Anspruch 1, **dadurch gekennzeichnet, dass** man die nach Schritt c) erhaltene mono-carboxyfunktionalisierten Dialkylphosphinsäure, deren Salz oder Ester (III) anschließend in einem Schritt d) mit Metallverbindungen von Mg, Ca, Al, Sb, Sn, Ge, Ti, Fe, Zr, Zn, Ce, Bi, Sr, Mn, Li, Na, K und/oder einer protonierte Stickstoffbase zu den entsprechenden mono-carboxyfunktionalisierten Dialkyl-phosphinsäuresalzen (III) dieser Metalle und/oder einer Stickstoffverbindung umsetzt.

3. Verfahren nach Anspruch 1 oder 2, **dadurch gekennzeichnet, dass** R¹, R², R³, R⁴, R⁵, R⁶, R⁷ gleich oder verschieden sind und, unabhängig voneinander H, Methyl, Ethyl, n-Propyl, Isopropyl, n-Butyl, Isobutyl, tert. Butyl und/oder Phenyl bedeuten.

4. Verfahren nach einem oder mehreren der Ansprüche 1 bis 3, **dadurch gekennzeichnet, dass** X und Y gleich oder verschieden sind und jeweils H, Ca, Mg, Al, Zn, Ti, Fe, Ce, Methyl, Ethyl, n-Propyl, Isopropyl, n-Butyl, Isobutyl, tert. Butyl, Phenyl, Ethylenglykol, Propylglykol, Butylglykol, Pentylglykol, Hexylglykol, Allyl und/oder Glycerin bedeuten.

5. Verfahren nach einem oder mehreren der Ansprüche 1 bis 4, **dadurch gekennzeichnet, dass** es sich bei den Übergangsmetallen und/oder Übergangsmetallverbindungen um solche aus der ersten, siebten und achten Nebengruppe handelt.

6. Verfahren nach einem oder mehreren der Ansprüche 1 bis 5, **dadurch gekennzeichnet, dass** es sich bei den Übergangsmetallen und/oder Übergangsmetallverbindungen um Rhodium, Nickel, Palladium, Platin, Ruthenium und/oder Gold handelt.

7. Verfahren nach einem oder mehreren der Ansprüche 1 bis 6, **dadurch gekennzeichnet, dass** es sich bei dem Katalysator B um Wasserstoffperoxid, Natriumperoxid, Lithiumperoxid, Kaliumpersulfat, Natriumpersulfat, Ammoniumpersulfat, Natriumperoxodisulfat, Kaliumperoxoborat, Peressigsäure, Benzoylperoxid, Di-t-butylperoxid und/oder Peroxodischwefelsäure und/oder um Azodiisobutyronitril, 2,2'-Azobis(2-amidinopropan)-dihydrochlorid und/oder 2,2'-Azobis(N,N'-dimethylen-isobutyramidin)-dihydrochlorid und/oder um Lithium, Lithiumhydrid, Lithium-aluminiumhydrid, Methyllithium, Butyllithium, t-Butyllithium, Lithiumdiisopropylamid, Natrium, Natriumhydrid, Natriumborhydrid, Natriummethanolat, Natriumethanolat oder Natriumbutylat, Kaliummethanolat, Kaliumethanolat und/oder Kaliumbutylat handelt.

8. Verfahren nach Anspruch 1 bis 7, **dadurch gekennzeichnet, dass** es sich bei den Oxidationsmitteln um Kaliumpermanganat, Braunstein, Chromtrioxid, Kaliumdichromat, Pyridindichromat, Pyridinchlorchromat, Collins-Reagenz, Jones-Reagenz, Corey-Gilman-Ganem-Reagenz, (Dess-Martin-)Periodinan, o-lodoxybenzoesäure, Rutheniumtetroxid, Rutheniumdioxid, Tetra-n-propyl-perruthenat, Rutheniumtrichlorid/Natriumperiodat, Rutheniumdioxid/Natriumperiodat, Chlor, Hypochlorit und Peroxoverbindungen handelt.

9. Verfahren nach einem oder mehreren der Ansprüche 1 bis 8, **dadurch gekennzeichnet, dass** es sich bei den Allylalkoholderivaten (V) um 2-Propen-1-ol, 2-Methyl-2-propen-1-ol, 2-isobutyl-2-propen-1-ol, 2-(Trimethylsilyl)-2-propen-1-ol, 3-Phenyl-2-propen-1-ol, 3-(Trimethylsilyl)-2-propen-1-ol, 3-(4-Hydroxy-3-methoxyphenyl)-2-propen-1-ol, 2-Methyl-3-phenyl-2-propen-1-ol, 2-Buten-1-ol, 2-Methyl-2-buten-1-ol, 3-(Trimethylsilyl)-2-buten-1-ol, 3-Methyl-2-buten-1-ol, 3-Phenyl-2-buten-1-ol, 3-(Trimethylsilyl)-2-buten-1-ol, 2-Methyl-3-phenyl-2-buten-1-ol, 2-Penten-1-ol, 2-Methyl-2-penten-1-ol, 2-(Trimethylsilyl)-2-penten-1-ol, 3-Methyl-2-penten-1-ol, 3-Phenyl-2-penten-1-ol, 4-Methyl-2-penten-1-ol und/oder 4-Phenyl-2-penten-1-ol handelt.

10. Verfahren nach einem oder mehreren der Ansprüche 1 bis 9, **dadurch gekennzeichnet, dass** es sich bei den Acroleinderivaten (V') um 2-Propenal, 2-Methyl-2-propenal, 2-Phenyl-2-propenal, 3-Phenyl-2-propenal, 2-Methyl-3-phenyl-2-propenal, 2-Butenal, 2-Methyl-2-butenal, 2-Phenyl-2-butenal, 3-Methyl-2-butenal, 2-Methyl-2-butenal, 2-Pentenal, 2-Methyl-2-pentenal, 2-Phenyl-2-pentenal, 4-Methyl-2-phenyl-2-pentenal und/oder 2,2-Dimethyl-4-pentenal handelt.

11. Verfahren nach einem oder mehreren der Ansprüche 1 bis 10, **dadurch gekennzeichnet, dass** es bei dem Alkohol der allgemeinen Formel M-OH um lineare oder verzweigte, gesättigte und ungesättigte, einwertige organische Alkohole mit einer Kohlenstoffkettenlänge von C₁-C₁₈ und es bei dem Alkohol der allgemeinen Formel M'-OH um lineare oder verzweigte, gesättigte und ungesättigte, mehrwertige organische Alkohole mit einer Kohlenstoffkettenlänge von C₁-C₁₈ handelt.

12. Herstellung von mono-carboxyfunktionalisierten Dialkylphosphinsäuren, -estern und -salzen, nach einem oder mehreren der Ansprüche 1 bis 11 und anschließende Verwendung dieser Produkte als Binder, als Vernetzer bzw. Beschleuniger beim Aushärten von Epoxyharzen, Polyurethanen und ungesättigten Polyesterharzen, als Polymerstabilisatoren, als Pflanzenschutzmittel, als Sequestrierungsmittel, als Mineralöl-Additiv, als Korrosionsschutzmittel, in Wasch- und Reinigungsmittelanwendungen und in Elektronikanwendungen.

## Claims

1. A method for producing monocarboxy-functionalized dialkylphosphinic acids, esters and salts, which comprises
a) reacting a phosphinic acid source (I) 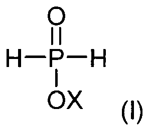 with olefins (IV) 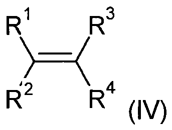 in the presence of a catalyst A to form an alkylphosphonous acid, salt or ester (II) 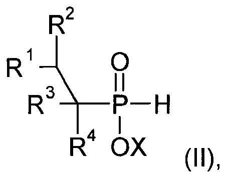
b) reacting the resulting alkyllphosphonous acid, salt or ester (II) with an allyl alcohol (V) and/or acrolein (V') 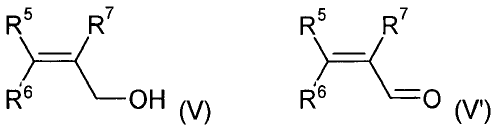 in the presence of a catalyst B to form the monofunctionalized dialkylphosphinic acid derivative (VI) and/or (VI') 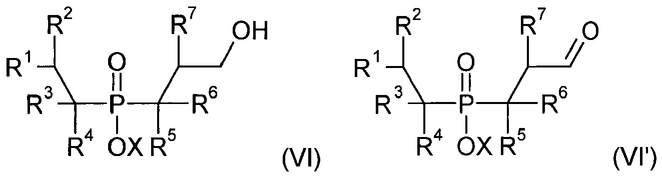 and
c) reacting the monofunctionalized dialkylphosphinic acid derivative (VI) and/or (VI') with an oxidant or with an oxidant and water or in the presence of a catalyst C with oxygen and water to form the monocarboxy-functionalized dialkylphosphinic acid derivative (III) 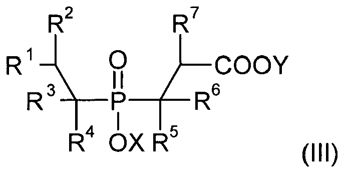 or esterifying the alkylphosphonous acid, salt or ester (II) obtained after step a) and/or the monofunctionalized dialkylphosphinic acid, salt or ester (VI) and/or (VI') obtained after step b) and/or the monocarboxy-functionalized dialkylphosphinic acid, salt or ester (III) obtained after step c) and/or the particular resulting reaction solution thereof with an alkylene oxide or an alcohol M-OH and/or M'-OH, and subjecting the respectively resulting alkylphosphonous ester (II), monofunctionalized dialkylphosphinic ester (VI) and/or (VI'), and/or monocarboxyfunctionalized dialkylphosphinic ester (III) to the further reaction steps b) or c), where R¹, R², R³, R⁴, R⁵, R⁶, R⁷ are identical or different and are each independently H, C₁-C₁₈-alkyl, C₆-C₁₈-aryl, C₆-C₁₈-aralkyl, C₆-C₁₈-alkylaryl, CN, CHO, OC(O)CH₂CN, CH(OH)C₂H₅, CH₂CH(OH)CH₃, 9-anthracene, 2-pyrrolidone, (CH₂)ₘOH, (CH₂)ₘNH₂, (CH₂)ₘNCS, (CH₂)ₘNC(S)NH₂, (CH₂)ₘSH, (CH₂)ₘS-2-thiazoline, (CH₂)ₘSiMe₃, C(O)R⁸, CH=CH-R⁸, CH=CH-C(O)R⁸, where the groups C₆-C₁₈-aryl, C₆-C₁₈-aralkyl and C₆-C₁₈-alkylaryl may be substituted with -C(O)CH₃, OH, CH₂OH, NH₂, NO₂, OCH₃, SH and/or OC(O)CH₃ and where R⁸ is C₁-C₈-alkyl or C₆-C₁₈-aryl and m is an integer from 0 to 10 and X and Y are identical or different and are each independently H, C₁-C₁₈-alkyl, C₆-C₁₈-aryl, C₆-C₁₈-aralkyl, C₆-C₁₈-alkylaryl, (CH₂)ₖOH, CH₂-CHOH-CH₂OH, (CH₂)ₖO(CH₂)ₖH, (CH₂)ₖ-CH(OH)-(CH₂)ₖH, (CH₂-CH₂O)ₖH, (CH₂-C[CH₃]HO)ₖH, (CH₂-C[CH₃]HO)ₖ(CH₂-CH₂O)ₖH, (CH₂-CH₂O)ₖ(CH₂-C[CH₃]HO)H, (CH₂-CH₂O)ₖ-alkyl, (CH₂-C[CH₃]HO)ₖ-alkyl, (CH₂-C[CH₃]HO)ₖ(CH₂-CH₂O)ₖ-alkyl, (CH₂-CH₂O)ₖ(CH₂-C[CH₃]HO)O-alkyl, (CH₂)ₖ-CH=CH(CH₂)ₖH, (CH₂)ₖNH₂, (CH₂)ₖN[(CH₂)ₖH]₂, where k is an integer from 0 to 10, and/or Mg, Ca, Al, Sb, Sn, Ge, Ti, Fe, Zr, Zn, Ce, Bi, Sr, Mn, Cu, Ni, Li, Na, K, H and/or a protonated nitrogen base and the catalysts A and C comprise transition metals and/or transition metal compounds and/or catalyst systems composed of a transition metal and/or transition metal compound and at least one ligand, and the catalyst B comprises peroxide-forming compounds and/or peroxo compounds and/or comprises azo compounds and/or comprises alkali metals and/or alkaline earth metals, hydrides and/or alkoxides.

2. The method according to claim 1 wherein the monocarboxy-functionalized dialkylphosphinic acid, its salt or ester (III) obtained after step c) is subsequently reacted in a step d) with metal compounds of Mg, Ca, Al, Sb, Sn, Ge, Ti, Fe, Zr, Zn, Ce, Bi, Sr, Mn, Li, Na, K and/or a protonated nitrogen base to form the corresponding monocarboxy-functionalized dialkylphosphinic acid salts (III) of these metals and/or of a nitrogen compound.

3. The method according to claim 1 or 2 wherein R¹, R², R³, R⁴, R⁵, R⁶, R⁷ are identical or different and are each independently H, methyl, ethyl, n-propyl, isopropyl, n-butyl, isobutyl, tert-butyl and/or phenyl.

4. The method according to one or more of claims 1 to 3 wherein X and Y are identical or different and are each H, Ca, Mg, Al, Zn, Ti, Fe, Ce, methyl, ethyl, n-propyl, isopropyl, n-butyl, isobutyl, tert-butyl, phenyl, ethylene glycol, propyl glycol, butyl glycol, pentyl glycol, hexyl glycol, allyl and/or glycerol.

5. The method according to one or more of claims 1 to 4 wherein the transition metals and/or transition metal compounds comprise such from the first, seventh and eighth transition groups.

6. The method according to one or more of claims 1 to 5 wherein the transition metals and/or transition metal compounds comprise rhodium, nickel, palladium, platinum, ruthenium and/or gold.

7. The method according to one or more of claims 1 to 6 wherein the catalyst B comprises hydrogen peroxide, sodium peroxide, lithium peroxide, potassium persulfate, sodium persulfate, ammonium persulfate, sodium peroxodisulfate, potassium peroxoborate, peracetic acid, benzoyl peroxide, di-t-butyl peroxide and/or peroxodisulfuric acid and/or comprises azobisisobutyronitrile, 2,2'-azobis(2-amidinopropane) dihydrochloride and/or 2,2'azobis(N,N'dimethylene-isobutyramidine) dihydrochloride and/or comprises lithium, lithium hydride, lithium aluminum hydride, methyllithium, butyllithium, t-butyllithium, lithium diisopropylamide, sodium, sodium hydride, sodium borohydride, sodium methoxide, sodium ethoxide, sodium butoxide, potassium methoxide, potassium ethoxide and/or potassium butoxide.

8. The method according to claim 1 or 2 or 3 or 4 or 5 or 6 or 7 wherein the oxidizing agents comprise potassium permanganate, manganese oxide, chromium trioxide, potassium dichromate, pyridine dichromate, pyridine chlorochromate, Collins reagent, Jones reagent, Corey-Gilman-Ganem reagent, (Dess-Martin) periodinane, o-iodoxybenzoic acid, ruthenium tetroxide, ruthenium dioxide, tetra-n-propyl perruthenate, ruthenium trichloride/sodium periodate, ruthenium dioxide/sodium periodate, chlorine, hypochlorite and peroxo compounds.

9. The method according to one or more of claims 1 to 8 wherein the allyl alcohol derivatives (V) comprise 2-propen-1-ol, 2-methyl-2-propen-1-ol, 2-isobutyl-2-propen-1-ol, 2-(trimethylsilyl)-2-propen-1-ol, 3-phenyl-2-propen-1-ol, 3-(trimethylsilyl)-2-propen-1-ol, 3-(4-hydroxy-3-methoxyphenyl)-2-propen-1-ol, 2-methyl-3-phenyl-2-propen-1-ol, 2-buten-1-ol, 2-methyl-2-buten-1-ol, 3-(trimethylsilyl)-2-buten-1-ol, 3-methyl-2-buten-1-ol, 3-phenyl-2-buten-1-ol, 3-(trimethylsilyl)-2-buten-1-ol, 2-methyl-3-phenyl-2-buten-1-ol, 2-penten-1-ol, 2-methyl-2-penten-1-ol, 2-(trimethylsilyl)-2-penten-1-ol, 3-methyl-2-penten-1-ol, 3-phenyl-2-penten-1-ol, 4-methyl-2-penten-1-ol and/or 4-phenyl-2-penten-1-ol.

10. The method according to one or more of claims 1 to 9 wherein the acrolein derivatives (V') comprise 2-propenal, 2-methyl-2-propenal, 2-phenyl-2-propenal, 3-phenyl-2-propenal, 2-methyl-3-phenyl-2-propenal, 2-butenal, 2-methyl-2-butenal, 2-phenyl-2-butenal, 3-methyl-2-butenal, 2-methyl-2-butenal, 2-pentenal, 2-methyl-2-pentenal, 2-phenyl-2-pentenal, 4-methyl-2-phenyl-2-pentenal and/or 2,2-dimethyl-4-pentenal.

11. The method according to one of claims 1 to 10 wherein the alcohol of the general formula M-OH comprises linear or branched, saturated and unsaturated, monohydric organic alcohols having a carbon chain length of C₁-C₁₈ and the alcohol of the general formula M'-OH comprises linear or branched, saturated and unsaturated polyhydric organic alcohols having a carbon chain length of C₁-C₁₈.

12. The preparation of monocarboxy-functionalized dialkylphosphinic acids, esters and salts according to one or more of claims 1 to 11 and subsequent use of these products as a binder, as a crosslinker or accelerant to cure epoxy resins, polyurethanes and unsaturated polyester resins, as polymer stabilizers, as crop protection agents, as a sequestrant, as a mineral oil additive, as a corrosion control agent, in washing and cleaning applications and in electronic applications.

## Revendications

1. Procédé pour la préparation d'acides, d'esters et de sels d'acides dialkylphosphoniques monofonctionnalisés par carboxy, **caractérisé en ce que**
a) on transforme une source d'acide phosphinique (I) 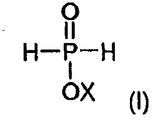 avec des oléfines (IV) 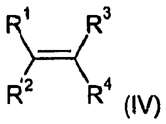 en présence d'un catalyseur A en acide alkylphosphoneux, son sel ou son ester (II) 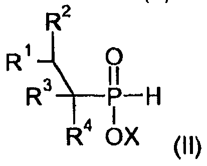
b) on transforme l'acide alkylphosphoneux ainsi formé, son sel ou son ester (II) avec un alcool allylique (V) et/ou une acroléine (V') 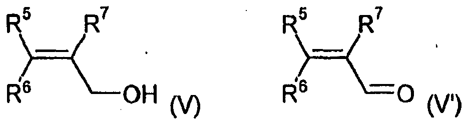 en présence d'un catalyseur B en dérivé d'acide dialkylphosphinique (VI) et/ou (VI') monofonctionnalisé 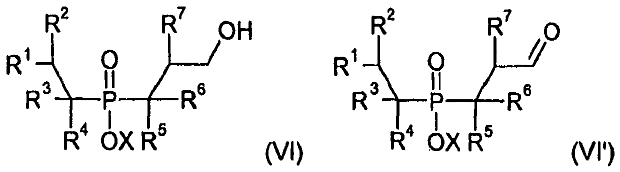 et
c) on transforme le dérivé d'acide dialkylphosphinique (VI) et/ou (VI') monofonctionnalisé
avec un oxydant ou avec un oxydant et de l'eau ou en présence d'un catalyseur C avec de l'oxygène et de l'eau en dérivé d'acide dialkylphosphinique (III) monofonctionnalisé par carboxy 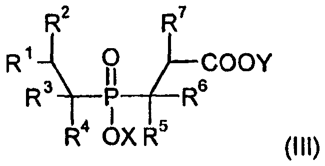 ou on estérifie l'acide alkylphosphoneux obtenu après l'étape a), son sel ou son ester (II) et/ou l'acide dialkylphosphinique monofonctionnalisé obtenu après l'étape b), son sel ou son ester (VI) et/ou (VI') et/ou l'acide dialkylphosphinique monofonctionnalisé par carboxy obtenu après l'étape c), son sel ou son ester (III) et/ou à chaque fois la solution réactionnelle résultante de ceux-ci avec un oxyde d'alkylène ou un alcool M-OH et/ou M'-OH, et on soumet l'ester de l'acide alkylphosphoneux (II), l'ester de l'acide dialkylphosphinique monofonctionnalisé (VI) et/ou (VI') et/ou l'acide dialkylphosphinique (III) monofonctionnalisé par carboxy à chaque fois obtenu aux autres étapes de réaction b) ou c), R¹, R², R³, R⁴, R⁵, R⁶, R⁷ étant identiques ou différents et signifiant, indépendamment l'un de l'autre, H, C₁-C₁₈-alkyle, C₆-C₁₈-aryle, C₆-C₁₈-aralkyle, C₆-C₁₈-alkyl-aryle, CN, CHO, OC(O)CH₂CN, CH(OH)C₂H₅, CH₂CH(OH)CH₃, 9-anthracène, 2-pyrrolidone, (CH₂)ₘOH, (CH₂)ₘNH₂, (CH₂)ₘNCS, (CH₂)ₘNC(S)NH₂, (CH₂)ₘSH, (CH₂)ₘS-2-thiazoline, (CH₂)ₘSiMe₃, C(O)R⁸, CH=CH-R⁸, CH=CH-C(O)R⁸, les groupes C₆-C₁₈-aryle, C₆-C₁₈-aralkyle et C₆-C₁₈-alkyl-aryle pouvant être substitués par -C(O)CH₃, OH, CH₂OH, NH₂, NO₂, OCH₃, SH et/ou OC(O)CH₃ et R⁸ représentant C₁-C₈-alkyle ou C₆-C₁₈-aryle et m valant un nombre entier de 0 à 10 et X et Y étant identiques ou différents et représentant, indépendamment l'un de l'autre, H, C₁-C₁₈-alkyle, C₆-C₁₈-aryle, C₆-C₁₈-aralkyle, C₆-C₁₈-alkyl-aryle, (CH₂)ₖOH, CH₂-CHOH-CH₂OH, (CH₂)ₖO(CH₂)ₖH, (CH₂)ₖ-CH(OH)-(CH₂)ₖH, (CH₂-CH₂O)ₖH, (CH₂-C[CH₃]HO)ₖH, (CH₂-C[CH₃]HO)ₖ(CH₂-CH₂O)ₖH, (CH₂-CH₂O)ₖ(CH₂-C[CH₃]HO)H, (CH₂-CH₂O)ₖ-alkyle, (CH₂-C[CH₃]HO)ₖ-alkyle, (CH₂-C[CH₃]HO)ₖ(CH₂-CH₂O)ₖ-alkyle, (CH₂-CH₂O)ₖ(CH₂-C[CH₃]HO)O-alkyle, (CH₂)ₖ-CH=CH(CH₂)ₖH, (CH₂)ₖNH₂, (CH₂)ₖN[(CH₂)ₖH]₂, k valant un nombre entier de 0 à 10 et/ou représentant Mg, Ca, Al, Sb, Sn, Ge, Ti, Fe, Zr, Zn, Ce, Bi, Sr, Mn, Cu, Ni, Li, Na, K, H et/ou une base azotée protonée et où il s'agit, pour les catalyseurs A et C de métaux de transition et/ou de composés de métaux de transition et/ou de systèmes catalytiques, qui sont composés d'un métal de transition et/ou d'un composé de métal de transition et d'au moins un ligand et où il s'agit pour le catalyseur B de composés formant des peroxydes et/ou de composés peroxo et/ou de composés azo et/ou de métaux, d'hydrures et/ou d'alcoolates de métal alcalin et/ou alcalino-terreux.

2. Procédé selon la revendication 1, **caractérisé en ce qu'**on transforme ensuite l'acide dialkylphosphonique monofonctionnalisé par carboxy obtenu après l'étape c), son sel ou son ester (III) dans une étape d) avec des composés métalliques de Mg, Ca, Al, Sb, Sn, Ge, Ti, Fe, Zr, Zn, Ce, Bi, Sr, Mn, Li, Na, K et/ou une base azotée protonée en sels d'acide dialkylphosphonique monofonctionnalisé par carboxy correspondants (III) de ces métaux et/ou d'un composé azoté.

3. Procédé selon la revendication 1 ou 2, **caractérisé en ce que** R¹, R², R³, R⁴, R⁵, R⁶, R⁷ sont identiques ou différents et signifient indépendamment l'un de l'autre, H, méthyle, éthyle, n-propyle, iso-propyle, n-butyle, iso-butyle, tert-butyle et/ou phényle.

4. Procédé selon l'une ou plusieurs des revendications 1 à 3, **caractérisé en ce que** X et Y sont identiques ou différents et représentent à chaque fois H, Ca, Mg, Al, Zn, Ti, Fe, Ce, méthyle, éthyle, n-propyle, iso-propyle, n-butyle, isobutyle, tert-butyle, phényle, éthylèneglycol, propylglycol, butylglycol, pentylglycol, hexylglycol, allyle et/ou glycérol.

5. Procédé selon l'une ou plusieurs des revendications 1 à 4, **caractérisé en ce qu'**il s'agit, pour les métaux de transition et/ou les composés de métaux de transition de ceux du premier, septième et huitième groupe secondaire.

6. Procédé selon l'une ou plusieurs des revendications 1 à 5, **caractérisé en ce qu'**il s'agit, pour les métaux de transition et/ou les composés de métaux de transition, de rhodium, de nickel, de palladium, de platine, de ruthénium et/ou d'or.

7. Procédé selon l'une ou plusieurs des revendications 1 à 6, **caractérisé en ce qu'**il s'agit, pour le catalyseur B, de peroxyde d'hydrogène, de peroxyde de sodium, de peroxyde de lithium, de persulfate de potassium, de persulfate de sodium, de persulfate d'ammonium, de peroxodisulfate de sodium, de peroxoborate de potassium, d'acide peracétique, de peroxyde de benzoyle, de peroxyde de di-t-butyle et/ou d'acide peroxodisulfurique et/ou d'azodiisobutyronitrile, de dichlorhydrate de 2,2'-azobis(2-amidinopropane) et/ou de dichlorhydrate de 2,2'-azobis(N,N'-diméthylène-isobutyramidine) et/ou de lithium, d'hydrure de lithium, d'hydrure de lithium-aluminium, de méthyllithium, de butyllithium, de t-butyllithium, de diisopropylamide de lithium, de sodium, d'hydrure de sodium, de borohydrure de sodium, de méthanolate de sodium, d'éthanolate de sodium ou de butylate de sodium, de méthanolate de potassium, d'éthanolate de potassium et/ou de butylate de potassium.

8. Procédé selon la revendication 1 à 7, **caractérisé en ce qu'**il s'agit, pour les oxydants, de permanganate de potassium, de dioxyde de manganèse, de trioxyde de chrome, de dichromate de potassium, de dichromate de pyridine, de chlorochromate de pyridine, de réactif de Collins, de réactif de Jones, de réactif de Corey-Gilman-Ganem, de periodonane (de Dess-Martin), d'acide o-iodoxybenzoïque, de tétroxyde de ruthénium, de dioxyde de ruthénium, de perruthénate de tétra-n-propyle, de trichlorure de ruthénium/periodate de sodium, de dioxyde de ruthénium/periodate de sodium, de chlore, d'hypochlorite et de composés peroxo.

9. Procédé selon l'une ou plusieurs des revendications 1 à 8, **caractérisé en ce qu'**il s'agit, pour les dérivés d'alcool allylique (V) de 2-propén-1-ol, de 2-méthyl-2-propén-1-ol, de 2-isobutyl-2-propén-1-ol, de 2-(triméthylsilyl)-2-propén-1-ol, de 3-phényl-2-propén-1-ol, de 3-(triméthylsilyl)-2-propén-1-ol, de 3-(4-hydroxy-3-méthoxyphényl)-2-propén-1-ol, de 2-méthyl-3-phényl-2-propén-1-ol, de 2-butén-1-ol, de 2-méthyl-2-butén-1-ol, de 3-(triméthylsilyl)-2-butén-1-ol, de 3-méthyl-2-butén-1-ol, de 3-phényl-2-butén-1-ol, de 3-(triméthylsilyl)-2-butén-1-ol, de 2-méthyl-3-phényl-2-butén-1-ol, de 2-pentén-1-ol, de 2-méthyl-2-pentén-1-ol, de 2-(triméthylsilyl)-2-pentén-1-ol, de 3-méthyl-2-pentén-1-ol, de 3-phényl-2-pentén-1-ol, de 4-méthyl-2-pentén-1-ol et/ou de 4-phényl-2-pentén-1-ol.

10. Procédé selon l'une ou plusieurs des revendications 1 à 9, **caractérisé en ce qu'**il s'agit, pour les dérivés d'acroléine (V'), de 2-propénal, de 2-méthyl-2-propénal, de 2-phényl-2-propénal, de 3-phényl-2-propénal, de 2-méthyl-3-phényl-2-propénal, de 2-buténal, de 2-méthyl-2-buténal, de 2-phényl-2-buténal, de 3-méthyl-2-buténal, de 2-méthyl-2-buténal, de 2-penténal, de 2-méthyl-2-penténal, de 2-phényl-2-penténal, de 4-méthyl-2-phényl-2-penténal et/ou de 2,2-diméthyl-4-penténal.

11. Procédé selon l'une ou plusieurs des revendications 1 à 10, **caractérisé en ce qu'**il s'agit, pour l'alcool de formule générale M-OH, d'alcools organiques linéaires ou ramifiés, saturés et insaturés, monovalents, présentant une longueur de chaîne carbonée de C₁-C₁₈ et il s'agit, pour l'alcool de formule générale M'-OH, d'alcools organiques linéaires ou ramifiés, saturés et insaturés, polyvalents présentant une longueur de chaîne carbonée de C₁-C₁₈.

12. Préparation d'acides, d'esters et de sels d'acides dialkylphosphoniques monofonctionnalisés par carboxy selon l'une ou plusieurs des revendications 1 à 11 et utilisation consécutive de ces produits comme liant, réticulant ou, selon le cas, accélérateur lors du durcissement de résines époxy, de polyuréthanes et de résines de polyester insaturées, comme stabilisants de polymères, comme agents de phytoprotection, comme agents de séquestration, comme additif d'huile minérale, comme agent de protection contre la corrosion, dans des utilisations d'agents de lavage et de nettoyage et dans des utilisations électroniques.
